# EUROPEAN PATENT APPLICATION

(11) **EP 4 776 237 A1**
(43) Date of publication of application: **15.07.2026**
(21) Application number: 26150705.7
(22) Date of filing: 08.01.2026
(51) Int. Cl.: G06V 10/25, G06V 40/10

(54) **HAND AND INSTRUMENT DETECTION FOR AUTOMATIC INSTRUMENT CONFIGURATION AND CONTROL**

(30) Priority: 10.01.2025 US 202563743790 P
(71) Applicant: Stryker European Operations Limited, Carrigtwohill, Co. Cork T45 HX08 (IE)
(72) Inventor: KUMAR, Anup, Gurugram (IN); LAUBENTHAL, Michael, Kalamazoo, 49001 (US); JAIN, Vikas, Gurugram (IN); PATEL, Vandit, Gurugram (IN)
(74) Representative: Röthinger, Rainer

(57) **Abstract**

A surgical system configured to control a surgical instrument along with methods for controlling the surgical system are provided. The surgical system includes a camera and a control system coupled to the camera and the surgical instrument. The control system is configured to receive video data from the camera, detect a hand of a user within the video data, identify a sub-region of the video data that includes the hand of the user, track the hand in the sub-region of the video data, detect a presence of the surgical instrument in the sub-region of the video data, determine at least one characteristic of the surgical instrument in response to detecting the presence of the surgical instrument in the sub-region of the video data, and control the surgical instrument based on the at least one characteristic of the surgical instrument.

## Description

### RELATED APPLICATIONS

The subject application claims priority to and all the benefits of United States Provisional Patent Application No. 63/743,790, filed on January 10, 2025, the entire contents of which are expressly incorporated herein by reference.

### BACKGROUND

Modern surgical operations often rely on surgical systems that involve multiple surgical instruments, each instrument being designed to handle specific functions in the operation room. For example, a surgery may necessitate a cutting instrument for cutting tissue at a surgical site, a suction instrument for drawing smoke away from the surgical site, and a drilling instrument for placing implants, among others. The instruments may be connected to the same surgical console and/or the same control device, such as a foot pedal, such that one console and one control device is used to control each instrument at different times or at the same time. Alternatively, the instruments may be connected to their own respective surgical console and control device. When using such a surgical system, a user must often switch between the different surgical instruments and control devices depending on a stage of a procedure being performed at the surgical site. At the same time, multiple users may be involved in the procedure, with each user carrying out a different portion of the procedure with a different surgical instrument and/or control device. For example, a first user may start a procedure using the cutting instrument and a first foot pedal to control the cutting instrument. Later on, the first user may put the cutting instrument down, pick up the suction instrument, and start using the first foot pedal to control the surgical suction instrument. At the same time, a second user may pick up the cutting instrument and begin using the second foot pedal to control the cutting instrument. In order to do so, at least one of the users may be required to manually reconfigure the surgical system. This can be a complicated and time consuming process, which can lead to mistakes and an unsatisfactory user experience.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical system is provided, comprising: a camera configured to capture video data; and a control system operatively coupled to the camera and a surgical instrument, the control system configured to: process the video data to detect a hand of a user; identify a region of interest in the video data associated with the hand; detect a presence of the surgical instrument in the region of interest; determine, within the region of interest, an action of the hand relative to the surgical instrument; and control the surgical instrument based on the determined action.

According to a second aspect, a surgical system is provided, comprising: a camera configured to capture video data; and a control system operatively coupled to the camera and a surgical instrument, the control system configured to: process the video data to detect a hand of a user; identify a region of interest in the video data associated with the hand; detect a presence of the surgical instrument in the region of interest and identify a physical characteristic of the surgical instrument; and control the surgical instrument based on the identification of the physical characteristic.

According to a third aspect, a surgical system is provided, comprising: a camera configured to capture video data; and a control system operatively coupled to the camera and a surgical instrument, the control system configured to: process the video data to detect a hand of a user; identify a region of interest in the video data associated with the hand; detect a presence of the surgical instrument in the region of interest and identify a procedural context characteristic of the surgical instrument; and control the surgical instrument based on the identification of the procedural context characteristic.

According to a fourth aspect, a surgical system is provided, comprising: a camera configured to capture video data; and a control system operatively coupled to the camera and a surgical instrument, the control system configured to: process the video data to detect a hand of a user; identify a region of interest in the video data associated with the hand; detect a presence of the surgical instrument in the region of interest and identify a hand interaction context characteristic relating to a context of interaction between the hand and the surgical instrument; and control the surgical instrument based on the identification of the hand interaction context.

According to a fifth aspect, a surgical system is provided, the surgical system is configured to control a surgical instrument, the surgical system comprising: a camera; and a control system coupled to the camera and the surgical instrument, wherein the control system is configured to: receive video data from the camera, detect a hand of a user within the video data, identify a sub-region of the video data that includes the hand of the user, track the hand in the sub-region of the video data, detect a presence of the surgical instrument in the sub-region of the video data, determine at least one characteristic of the surgical instrument in response to detecting the presence of the surgical instrument in the sub-region of the video data, and control the surgical instrument based on the at least one characteristic of the surgical instrument.

According to a sixth aspect, a surgical system is provided, the surgical system is configured to control a surgical instrument, the surgical system comprising: a camera; and a control system coupled to the camera and the surgical instrument, wherein the control system is configured to: receive video data from a camera, apply a hand detection algorithm to the video data to detect a hand of a user within the video data and estimate a hand region of the video data, track the hand in the hand region of the video data, apply an instrument-in-hand detection algorithm to the video data, determine at least one characteristic of the surgical instrument based on an output of the instrument-in-hand detection algorithm, and control the surgical instrument based on the at least one characteristic of the surgical instrument.

According to a seventh aspect, a surgical system is provided for tracking a hand of a user, the system comprising a first camera and a second camera and a control system configured to: capture a first image with the first camera, extract a first set of hand features associated with the hand of the user from the first image, create a hand tracking model based on the first set of hand features, track the hand with the first camera using the hand tracking model, capture a second image with the second camera, extract a second set of hand features associated with the hand of the user from the second image, modify the hand tracking model based on the second set of hand features to create an updated hand tracking model, and track the hand with at least one of the first camera and the second camera using the updated hand tracking model.

According to an eight aspect, a surgical system is provided for tracking a hand of a user, the system comprising a first camera and a second camera and a control system configured to: capture a first image with the first camera, extract a first set of hand features associated with the hand of the user from the first image, create a hand tracking model based on the first set of hand features, track the hand with the first camera using the hand tracking model, capture a second image with the first camera, detect the hand of the user in the second image, determine a tracking quality associated with the first camera by comparing the hand tracking model to the hand of the user in the second image, and track the hand with the second camera using the hand tracking model in response to the tracking quality being below a threshold quality.

According to a ninth aspect, a surgical system is provided for tracking a hand of a user, the surgical system comprising a camera, a sensor unit coupled to the user, and a control system configured to: capture a first image with the camera, extract a first set of hand features associated with the hand of the user from the first image, create a hand tracking model based on the first set of hand features, receive sensor data from the sensor unit, modify the hand tracking model based on the sensor data to create an updated hand tracking model, and track the hand with the camera using the updated hand tracking model.

A computer-implemented method is provided of operating the surgical system, control system, or surgical instrument of any preceding aspect. A non-transitory computer readable medium or computer program product is provided of operating the surgical system, control system, or surgical instrument of any preceding aspect.

Any of the above aspects can be combined in part or in whole with any other aspect. Any of the above aspects, whether combined in part or in whole, can be further combined with any of the following implementations, fully or in part.

The video data may be processed prior to detecting the hand of the user. Detecting the hand of the user within the video data can include detecting a set of landmarks associated with the hand in the video data. Further, identifying the sub-region of the video data may include generating a bounding region surrounding the detected set of landmarks in the video data. A range of motion of the hand may be determined and the bounding region may be generated to surround the plurality of hand landmarks throughout the range of motion of the hand. The bounding region may also be generated to surround the plurality of hand landmarks and a margin area surrounding the plurality of hand landmarks.

The at least one characteristic of the surgical instrument can include at least one of: (a) a physical characteristic including a geometry, an appearance, or a physical configuration of the surgical instrument; (b) an operational characteristic including a status of operation, a mode of operation, or an operational parameter of the surgical instrument; (c) a procedural context characteristic including a type of the surgical instrument, an identification of the surgical instrument, an accessory attached thereto, an interaction of the surgical instrument with an anatomy, or a procedural stage in which the surgical instrument is used; and (d) a hand interaction context characteristic relating to an interaction of the hand with the surgical instrument, the hand interaction context characteristic including at least one of the surgical instrument being held by the hand, being held by a primary hand, being held by a secondary hand, being held by both primary and secondary hands, a position and/or orientation of the surgical instrument when held by the hand, a change of pose or movement pattern of the surgical instrument when held by the hand, a duration the surgical instrument is held by the hand, the surgical instrument not being held by any hand, a position and/or orientation of the surgical instrument when not held by the hand, a duration the surgical instrument is not held by any hand, an action applied to the surgical instrument by the hand; a picking up of the surgical instrument by the hand, a placing down of the surgical instrument by the hand, or a spatial relationship or proximity between the hand and the surgical instrument.

The control system controls the surgical instrument based on the at least one characteristic by being configured to perform one or more of: activate the surgical instrument; deactivate the surgical instrument; adjust operational parameters of the surgical instrument; configure a mode of operation of the surgical instrument; configure the surgical instrument based on user-specific preferences; link the surgical instrument to a control device that control the surgical instrument; and unlink the surgical instrument from a control device that control the surgical instrument.

A hand detection algorithm may be applied to the video data and a bounding region surrounding the sub-region of the video data may be generated. Further, the hand detection algorithm may detect the hand of the user and identifies the sub-region of the video data which includes the hand of the user, and generating the bounding region may include removing or disregarding portions of the video data which are outside of the bounding region. The surgical instrument may be identified in the video data, and the bounding region may be generated to surround the hand of the user and the identified surgical instrument. The bounding region may be generated to surround the hand, the identified surgical instrument, and a margin area surrounding the hand and the identified surgical instrument.

The system can determine if the surgical instrument is being used. The system can apply an instrument-in-hand detection algorithm to the video data. The instrument-in-hand detection algorithm can detect the surgical instrument and identify an instrument region of the video data that includes the surgical instrument. The system can identify a sub-region of the video data that includes both the hand region and the instrument region. The system can classify the sub-region based on a tracked pose of the hand and the detected presence of the surgical instrument in the sub-region. The system can control the surgical instrument based on a characteristic of the surgical instrument and the classification of the sub-region. The system can calculate a distance between the hand region and the instrument region. The system can classify the sub-region based on the calculated distance. The system can compare the distance to a predefined distance threshold and classify the sub-region based on the comparison. The system can identify a hand contour associated with the hand of the user and an instrument contour associated with the surgical instrument. The system can determine whether the hand contour is surrounded by the instrument contour and classify the sub-region based on that determination. The system can estimate a pose of the hand based on the hand region of the video data. The system can compare the estimated pose of the hand to a set of predefined hand poses and classify the sub-region based on the comparison. The system can classify the sub-region based on a weight distribution within the hand region.

The system can determine which of a plurality of detected hands is the primary hand of the user, can recognize the hand of the user as the primary hand, and can identify a sub-region of the video data that includes a primary hand region. The system can apply a primary hand detection algorithm to the video data to identify a first hand region that includes a first hand of the user, identify a second hand region that includes a second hand of the user, and select one of the first hand region or the second hand region as the primary hand region of the video data.

The system can score detected hand regions to determine which of multiple detected hands is the primary hand of the user. The system can define a reference point in the video data, determine a center of the first hand region, determine a center of the second hand region, calculate a first distance between the reference point and the center of the first hand region, calculate a second distance between the reference point and the center of the second hand region, and select one of the first hand region or the second hand region as the primary hand region by comparing the first distance to the second distance. The system can select the first hand region as the primary hand region if the first distance is less than the second distance, and select the second hand region as the primary hand region if the second distance is less than the first distance. The reference point can be based on at least one of a center of the video data, a location of the surgical instrument, a location of a patient, a location of a surgical site, a location of a group of surgical instruments, or a location of a surgical device. Alternatively, the system can calculate an area of the first hand region, calculate an area of the second hand region, assign a first score to the first hand region based on its area, assign a second score to the second hand region based on its area, and select one of the first hand region or the second hand region as the primary hand region by comparing the first score to the second score, where the scores can be directly related to the respective areas.

The system can score detected hand regions based on pose by calculating a pose of the first hand region, calculating a pose of the second hand region, assigning a first score to the first hand region based on its pose, assigning a second score to the second hand region based on its pose, and selecting one of the hand regions as the primary hand region by comparing the scores. The system can define a predefined hand pose and assign scores based on similarity between each hand region's pose and the predefined pose. The system can also score detected hand regions based on interaction patterns by detecting a first interaction pattern for the first hand region, detecting a second interaction pattern for the second hand region, and selecting the primary hand region by comparing the interaction patterns or by assigning scores based on those patterns and comparing the scores. The system can determine the primary hand region based on a comparison to known hands by determining a first set of hand features for the first hand region, determining a second set of hand features for the second hand region, and comparing these sets to known hand features stored in a database. The system can assign scores based on similarity between each set of hand features and the known set of hand features and select the primary hand region by comparing the scores.

The system can present detected hand regions to the user and allow the user to select which hand region corresponds to the primary hand by receiving a user input and selecting one of the hand regions based on that input. The system can segment the video data into a first set of pixels corresponding to the hand and a second set of pixels corresponding to the surgical instrument, and can generate a bounding region surrounding the first set of pixels and the second set of pixels, including a margin area around them. The system can identify a hand contour surrounding the hand in the video data, determine a convex hull of the hand contour, and estimate a pose of the hand by determining a pose of the convex hull, and can generate the bounding region to surround the convex hull and an additional margin area. The system can classify the sub-region of the video data based on a tracked pose of the hand and the detected presence of the surgical instrument in the sub-region, and can control the surgical instrument based on a characteristic of the surgical instrument and the classification of the sub-region.

The system can involve the pose of the hand in controlling the surgical instrument by detecting the pose of the hand in the sub-region of the video data, classifying the sub-region based on the detected pose, and controlling the surgical instrument based on a characteristic of the instrument and the classification. The system can classify the sub-region based on whether the detected pose is a grip pose, can classify the sub-region as instrument-in-hand when the detected pose includes the grip pose, and can classify the sub-region as instrument-kept-down when the detected pose does not include the grip pose.

The system can respond to occlusion of the surgical instrument from the camera by determining whether the instrument is occluded by the hand in the sub-region of the video data and can trigger a notification when the instrument is determined to be occluded by the hand.

The system can operate on video data that includes multiple image frames by detecting the hand of the user within each frame, identifying sub-regions that include the hand, detecting the presence of the surgical instrument in each sub-region, and determining at least one characteristic of the surgical instrument based on its presence. The system can estimate a pose of the hand in each sub-region, classify each sub-region according to the estimated pose and the presence of the surgical instrument, and control the surgical instrument based on the characteristic and the classification of at least one sub-region. The system can also determine a count of consecutive image frames classified as instrument-kept-down and deactivate the surgical instrument if the count exceeds a threshold.

The system can use video data from multiple cameras by receiving second video data from a second camera, detecting a hand of the user within the second video data, identifying a sub-region that includes the hand, and tracking the hand in that sub-region. The system can determine the presence of the surgical instrument in the sub-region of the second video data, determine at least one additional characteristic of the surgical instrument based on its presence, and control the surgical instrument based on that characteristic. The system can monitor tracking quality for each camera and switch between cameras by determining a first tracking confidence for the sub-region of the first video data, determining a second tracking confidence for the sub-region of the second video data, and tracking the hand based on the confidence values. The system can define a tracking confidence threshold, compare the confidence values to the threshold, track the hand in the first video data when the first confidence exceeds the threshold, and track the hand in the second video data when the second confidence exceeds the threshold. The system can also avoid occlusion issues by determining that the hand is occluded in the first video data and tracking the hand in the second video data when occlusion occurs in the first video data.

The system can improve hand tracking by using multiple cameras by extracting a first set of hand features from the first video data, creating a hand tracking model based on the first set of features, extracting a second set of hand features from the second video data, and modifying the hand tracking model based on the second set of features to create an updated model for tracking the hand with at least one of the cameras. The system can alternatively create a second hand tracking model based on the second set of features and track the hand in the sub-region of the second video data using the second model. The system can combine these approaches with tracking quality concepts by determining a first tracking confidence for the first camera by comparing the first model to the hand in the first video data, determining a second tracking confidence for the second camera by comparing the second model to the hand in the second video data, and tracking the hand based on the confidence values. The system can define a tracking confidence threshold, compare the confidence values to the threshold, track the hand in the first video data when the first confidence exceeds the threshold, and track the hand in the second video data when the second confidence exceeds the threshold.

The system can incorporate sensor data from wearable sensors coupled to the user into hand tracking by extracting a first set of hand features from the video data, creating a hand tracking model based on the first set of features, receiving sensor data from a sensor unit coupled to the user, modifying the hand tracking model based on the sensor data to create an updated model, and tracking the hand with the camera using the updated model. The system can also involve multiple cameras along with sensor data by receiving second video data from a second camera, detecting the hand in the second video data, identifying a sub-region that includes the hand, extracting a second set of hand features from the second video data, modifying the updated hand tracking model based on the second set of features, and tracking the hand with at least one of the cameras using the updated model.

The system can develop or deploy a hand tracking model to track the hand of the user and can involve multiple cameras in creating the model by extracting a first set of hand features from the first video data, creating a hand tracking model based on the first set of features, and tracking the hand with the first camera using the model. The system can capture a second image with the second camera, extract a second set of hand features from the second image, match at least one feature of the first set to at least one feature of the second set, modify the hand tracking model based on the second set to create an updated model, and track the hand with at least one of the cameras using the updated model. The matching can include identifying which features of the second set correspond to features of the first set, and this process can be combined with occlusion-handling steps described above.

The system can use a first camera to reduce computational load on a second camera by estimating the sub-region of the first video data based on the sub-region of the second video data and identifying the sub-region of the first video data from that estimate. The first camera can capture video data in a first modality and the second camera can capture video data in a second modality, where the first modality can be optical imaging and the second modality can be thermal imaging. The system can identify the sub-region of the first video data based on the sub-region of the second video data and a registration transform associated with the cameras. The system can also use multiple cameras when multiple users are present by receiving second video data from a second camera, detecting a hand of a second user, identifying a sub-region that includes the second user's hand, and tracking that hand in the sub-region. The system can detect the presence of the surgical instrument in the sub-region of the second video data, set operational parameters of the instrument in response, and activate the instrument based on those parameters when its presence is detected in the sub-region of the first video data. The system can identify the surgical instrument in the first video data, detect its presence in the second video data, identify the instrument in the second video data, determine whether the instrument is the same in both video data sets, and trigger a notification or instruct at least one user to present the instrument to a camera for further identification when a difference in identity is detected. The system can determine at least one characteristic of the surgical instrument based on a pose of the hand.

### BRIEF DESCRIPTION OF THE DRAWINGS

Advantages of the present disclosure will be readily appreciated as the same becomes better understood by reference to the following detailed description when considered in connection with the accompanying drawings.
Figure 1 is a perspective view of a surgical system including a navigation system, a surgical robot, and an instrument suite.
Figure 2 is a schematic view of the surgical system of Figure 1.
Figure 3 is a block diagram illustrating how data from various video sources is analyzed and used to configure and control elements of a surgical system.
Figure 4 is a flow diagram describing a hand detection algorithm according to one implementation.
Figures 5A-5E each include a flow diagram describing a primary hand detection algorithm according to one implementation.
Figures 6A-6D each include a flow diagram describing a bounding region algorithm according to one implementation.
Figures 7A-7E each include a flow diagram describing an instrument-in-hand detection (IIHD) algorithm according to one implementation.
Figures 8A-11B show an example of hand and instrument tracking for instrument control and configuration.
Figures 12-15 each include a flow diagram describing a multiple camera tracking method according to one implementation.
Figures 16-18 each include a flow diagram describing a method of automatically configuring and controlling an instrument according to one implementation.

### DETAILED DESCRIPTION

Aspects of the present disclosure generally relate to improved systems and methods for automatically configuring and controlling interconnected surgical instruments, surgical consoles, and control devices. An exemplary surgical system may include multiple surgical consoles, each having at least one surgical instrument and external control device *(e.g.,* a foot pedal) connected thereto. In order to provide a simplified and improved user experience, the systems and methods described herein are capable of automatically adjusting operational parameters of surgical instruments, automatically linking and unlinking external control devices and surgical instruments, and automatically enabling and disabling surgical instruments based on which instruments are picked up/set down, among other features. Each of these features are carried out without user input, but rather based on video and/or image data depicting elements of the surgical system along with the user(s) and/or patient.

### I. Example System Overview

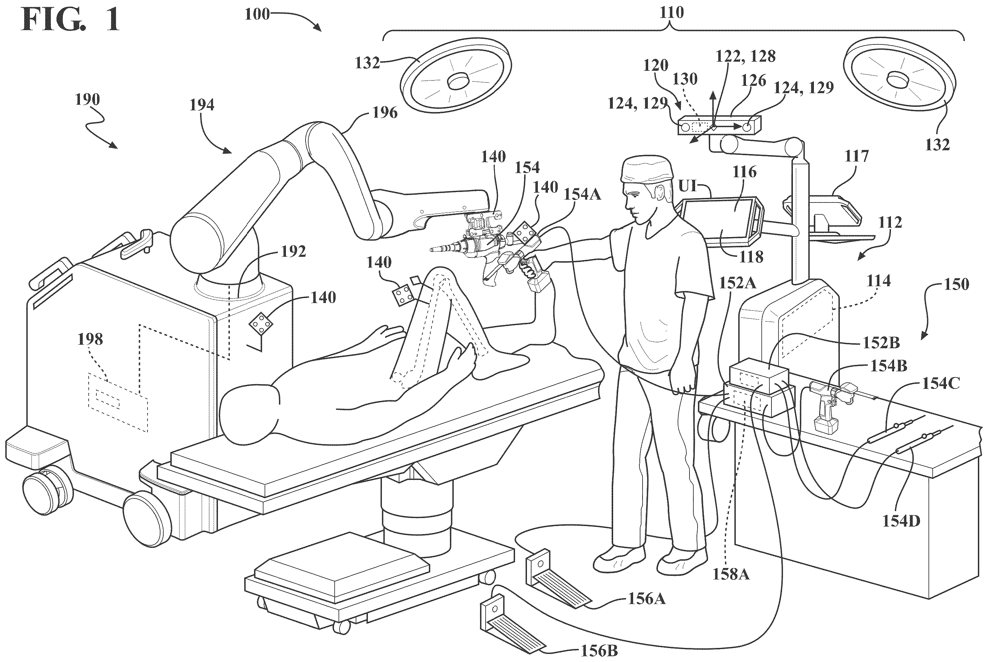

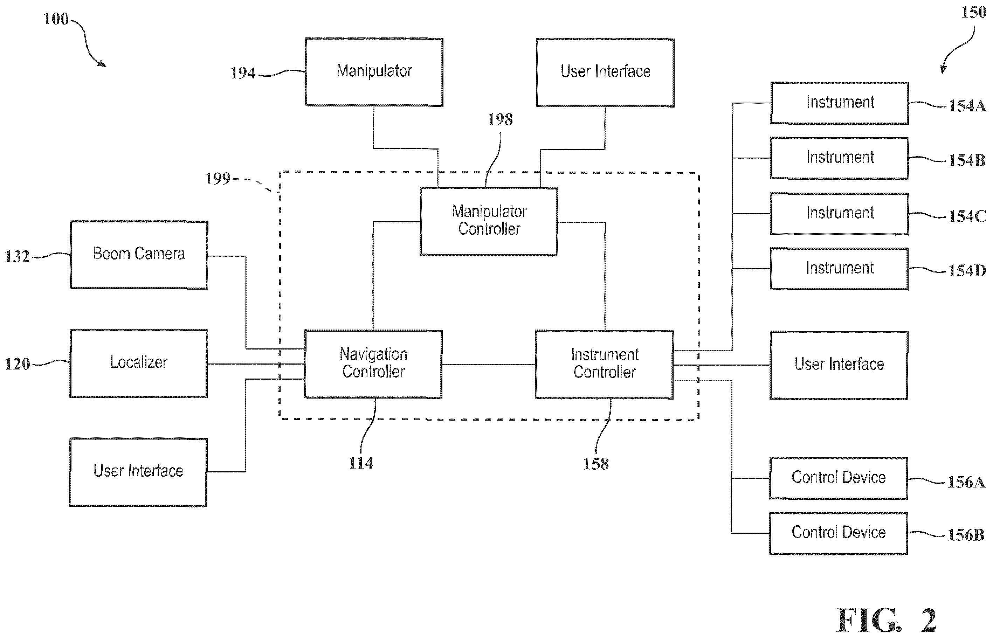

Referring to Figures 1 and 2, an example configuration of a surgical system 100 for assisting a user in performing a medical procedure on a patient is shown within an operating room. In the illustrated implementation, the surgical system 100 includes a surgical navigation system 110, a surgical instrument suite 150, and a surgical robot 190.

The surgical navigation system 110 is configured to track movement of various objects in the operating room, such as elements of the surgical instrument suite 150, the surgical robot 190, and/or the patient, among others. The surgical navigation system 110 tracks these objects for purposes including controlling the surgical instrument suite 150, controlling the surgical robot 190, displaying the relative positions and orientations of parts of the instrument suite 150 and the surgical robot 190 to the surgeon, and/or, in some cases, for controlling or constraining movement of elements of the instrument suit 150 and/or the surgical robot 190 relative to virtual cutting boundaries associated with the patient. Trackers 140 may be coupled to objects within the operating room, and the navigation system 110 may determine positions of the trackers 140 in order to track the objects associated with the trackers 140.

The surgical navigation system 110 may include a computer cart assembly 112 that houses a navigation controller 114 having a navigation interface in operative communication with the navigation controller 114. The navigation interface may include a first display 116 adapted to be situated outside of the sterile field and a second display 117 adapted to be situated inside the sterile field. The displays 116, 117 may be adjustably mounted to the computer cart assembly 112. At least one of the displays 116 may include a touch screen 118 that can be used to input information into the navigation controller 114 or otherwise select/control certain aspects of the navigation controller 114. Other input methods are also contemplated, such as a keyboard and mouse, gesture controls, or voice-activation. The displays 116, 117 can be implemented as head-mounted displays configured for extended reality or augmented reality and adapted to display any of the graphics or imagery described herein in manner that is overlaid or superimposed over real-world views or video.

Further, the navigation system 110 may include a localizer 120 in communication with the navigation controller 114. In the illustrated implementation, the localizer 120 is a multi-modality localizer and includes an optical (video) camera unit 122 and an infrared and/or near-infrared (NIR) sensor unit 124. The optical camera unit 122 includes one or more sensors 128 that are adapted to sense light in the visible spectrum and may be configured as a video camera or machine vision or computer vision system. The visible light sensors 128 are configured to detect color and or produce data that can be used to create depth maps. The infrared sensor unit 124 may include one or more sensors 129 that are adapted to sense light in the infrared or near-infrared spectrum. The localizer 120 may include illuminators that are configured to radiate infrared light into the surgical field to enable the infrared sensors 129 to detect reflected or backscatter radiation. An outer casing 126 houses the optical camera unit 122 and the infrared sensor unit 124. In some implementations, a camera controller 130 facilitates communication between the sensors 128, 129 and the navigation controller 114 through either a wired or a wireless connection (not shown). In other implementations, the sensors 128, 129 may communicate directly with the navigation controller 114. Processing of the signals from the visible light sensor(s) 128 and the IR sensor(s) 129 may occur at the navigation controller 114 for processing both navigation and machine vision information. One example of the navigation system 110 is described in U.S. Patent No. 9,008,757, entitled, "Navigation System Including Optical and Non-Optical Sensors," hereby incorporated by reference.

Additionally or alternatively, the surgical navigation system 110 may include at least one boom camera 132 coupled to a ceiling of the operating room and configured to be moved about the operating room while remaining fixed to the ceiling, or at least one head-mounted device (HMD) (not shown) coupled to the head of the user(s). One example of a suitable HMD is described in U.S. Application Publication No. 2024/0268892, entitled "Virtual Reality Surgical Systems And Methods Including Virtual Navigation," the entirety of which is incorporated by reference. The boom camera(s) 132 and/or the HMD may be in communication with the navigation controller 114. The localizer 120, boom camera(s) 132, and HMD may each include at least one of a machine vision camera, an optical camera, a thermal camera, and the like. The navigation system 110 may include any combination of the localizer 120, at least one boom camera 132, and at least one HMD. In the illustrated implementation, the navigation system 110 includes the localizer 120 and two boom cameras 132.

The surgical instrument suite 150 includes at least one surgical console 152, at least one surgical instrument 154, at least one control device 156, and at least one instrument controller 158. Through the present description, the surgical console(s) 152, the surgical instrument(s) 154, the control device(s) 156, and the instrument controller(s) 158 are primarily described in the singular for clarity. That said, any description of these elements 152, 154, 156, 158 should be understood to optionally include a plurality thereof 152, 154, 156, 158. In the illustrated implementation, the surgical instrument suit 150 includes a first surgical console 152A and a second surgical console 152B. The first console 152A has a first control device 156A, a first surgical instrument 154A, and a second surgical instrument 154B connected thereto. While the second console 152B is connected to a second control device 156B, a third surgical instrument 154C, and a fourth surgical instrument 154D.

The surgical console 152 is configured to control devices connected thereto, such as the surgical instruments 154 and the control devices 156. More specifically, the console 152 may set various operational parameters for the instruments 154, as well as associate the appropriate control devices 156 with the instruments 154. For example, depending on the surgical instruments 154 being used, the operational parameters may include torque level, end effector RPM, braking/acceleration of end effectors, oscillation frequency, and irrigation rates/suction force, among others. The operational parameters may be accessible to the user through a user interface presented on a display 119 on the console 152, through the user interface UI on the display 116 of the navigation system 110, or through any other user interface in communication with the console 152. As a result, the user may access any one of the user interfaces connected to the console 152, set the operational parameters as desired, and then use the surgical instrument 154 as the console 152 is controlling the instrument 154 in accordance with the operational parameters set by the user. One example of such a surgical console is the Core 2 Console, sold by Stryker and described in the International Application Publication No. 2015/021216 A1, entitled "System And Method For Driving An Ultrasonic Handpiece As A Function Of The Mechanical Impedance Of The Handpiece," the entirety of which is incorporated by reference. As described in the next section below, the system 100 may also automatically set the operational parameters for the surgical instrument 154 based on at least one of an identified user and an identified instrument.

The navigation system 110 may be used to track the surgical robot 190. In some implementations, the surgical robot 190 includes a base 192 and a manipulator 194 including a plurality of links and joints. The base 192 may be fixed to a point in the operating room, such as the operating table. Alternatively, the base 192 may be readily movable such that the surgical robot can be repositioned in the operating room. In one example, the surgical robot 190 can have a configuration like the robotic manipulator described in US Patent No. 10,327,849, entitled "Robotic System and Method for Backdriving the Same", the contents of which are hereby incorporated by reference in its entirety.

The surgical robot 190 may house a manipulator controller 198, or other type of control unit. The manipulator controller 198 may comprise one or more computers, or any other suitable form of controller that directs the motion of the manipulator 194 and/or the base 192. The manipulator controller 198 may have a central processing unit (CPU) and/or other processors, memory, and storage. The processors could include one or more processors to control operation of the manipulator 194. The processors can be any type of microprocessor, multi-processor, and/or multi-core processing system. The manipulator controller 198 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any implementation to a single processor. The surgical robot 190 may also comprise a user interface with one or more displays and/or input devices (*e.g*., push buttons, keyboard, mouse, microphone (voice-activation), gesture control devices, touchscreens, etc.).

The surgical robot 190 may be used to control the surgical instrument 154. In some implementations, the manipulator 194 and the instrument 154 may be arranged like that shown in U.S. Patent No. 9,566,121, filed on March 15, 2014, entitled, "End Effector of a Surgical Robotic Manipulator," hereby incorporated by reference. In other implementations, the surgical instrument 154 is attached to the manipulator 194 as shown in U.S. Pat. No. 9,119,655, issued Sep. 1, 2015, entitled, "Surgical Manipulator Capable of Controlling a Surgical Instrument in Multiple Modes", the disclosure of which is hereby incorporated by reference.

As shown in Figure 2, the various elements of the surgical system 100 may be in communication with one another. In particular, the navigation system 110 may communicate via the navigation controller 114, the surgical robot 190 may communicate via the manipulator controller 198, and the instrument suite 150 may communicate through at least one of the instrument controllers 158. The controllers 114, 158, 198 may together form a control system 199. In other implementations, the control system 199 may include its own computing device that is configured to communicate with the controllers 114, 158, 198. Further, the control system 199 may be a local computing system, or a cloud-based computing system. For ease of description, the control system 199 is herein described as handling communication between the elements of the system 100, as well as executing methods for controlling the same. It will be appreciated that the individual controllers 114, 158, 198 may instead control the associated elements, and portions of each method described herein may be carried out by the appropriate controller 114, 158, 198.

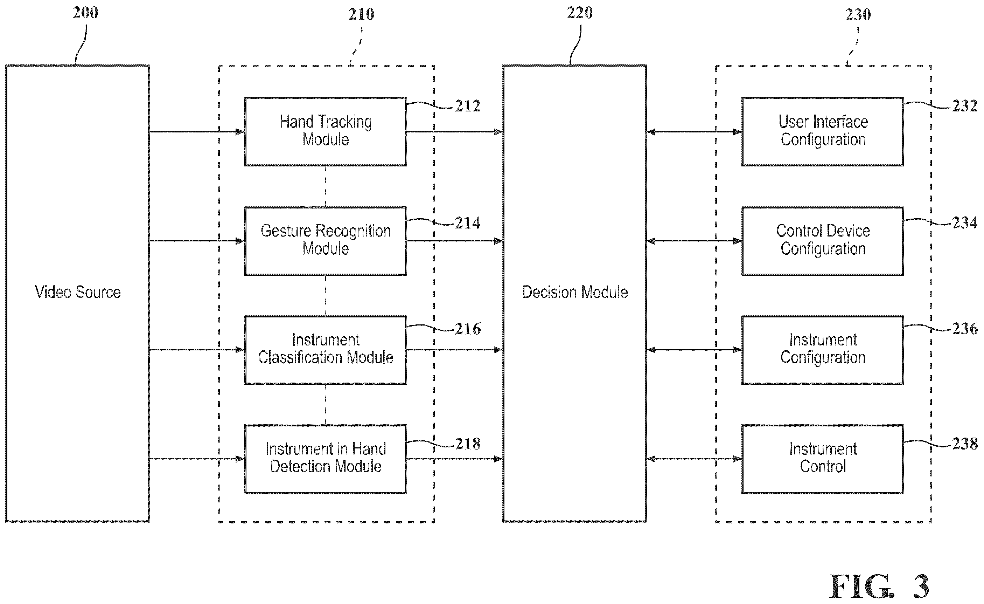

Referring to Figure 3, a block diagram illustrating how data from the various video/image sources is analyzed and used to configure and control elements of the surgical system 100 is depicted. The block diagram includes abstract representations of aspects of the surgical system, including a video source 200, an analysis module 210, a decision module 220, and a control module 230. In the illustrated implementation, the analysis module 210 includes a hand tracking module 212, a gesture recognition module 214, an instrument classification module 216, and an instrument-in-hand detection module 218. Further, the control module 230 may include a user interface configuration 232, a control device configuration 234, an instrument configuration 236, and/or instrument control 238. The video source 200 sends video data to the analysis module 210, the video data is analyzed by the analysis module 210 and converted into configuration and control instructions by the decision module 220, and the configuration and control instructions are then communicated to the appropriate elements of the surgical system 100 by the control module 230. In response, these elements automatically update their configuration and control parameters based on the video data from the video source 200. The abstract representations shown in Figure 3 may be carried out and received by specific elements of the surgical system 100. For example, the video source 200 may include at least one of the localizer 120 and the boom cameras 132, while the analysis, decision, and control modules 210, 220, 230 may be software modules executed by the control system 199. Further, by executing the software modules 210, 220, 230, the control system 199 may configure and control the surgical consoles 152, the surgical instruments 154, and the control devices 156.

In order to convert the video data captured by the video source 200 into configuration and control instructions, the analysis and decision modules 210, 220 may apply various video and data analysis techniques to the video data. More specifically, the hand tracking module 212 may analyze the video data to detect a hand of the user within the video data. Subsequently, the hand tracking module 212 may identify a sub-region of the video data that includes the hand of the user, as well as track the hand in the sub-region of the video data. The gesture recognition modules 214, on the other hand, may analyze the sub-region of the video data to estimate a pose of the hand of the user, compare the estimated pose of the hand to a set of predefined hand poses, and output a classification of the sub-region based on the comparison. Further, the instrument classification module 216 may analyze the sub-region of the video data to determine whether the surgical instrument 154 is present in the sub-region, and then output a classification of the surgical instrument 154 (and/or sub-region) based on identified features of the surgical instrument. Even further, the instrument-in-hand detection (IIHD) module 218 may determine if the surgical instrument 154 is being held by the hand of the user and output a classification of the sub-region based on this determination. The modules 212, 214, 216, 218 may communicate with one another as further described herein.

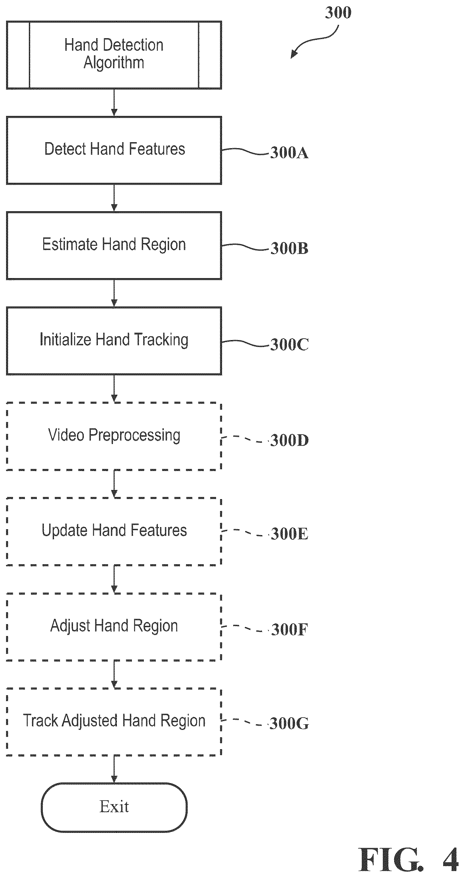

Although the gesture, instrument classification, and IIHD modules 214, 216, 218 may output classifications of the sub-region of the video data, the modules 214, 216, 218 may instead output data that can be used by the decision module 220 to classify the sub-region. Additionally or alternatively, the modules 212, 214, 216, 218 may carry out respective algorithms to perform the functions described herein. For example, the hand tracking module 212 may be configured to execute a hand detection algorithm, the gesture recognition module 214 may be configured to execute a gesture recognition algorithm, the instrument classification module 216 may be configured to execute an instrument classification algorithm, and the IIHD module 218 may be configured to execute an instrument-in-hand detection algorithm. Examples of algorithms carried out by the modules 212, 214, 216, 218 are shown in Figures 4-7E and described below.

### II. Hand and Instrument Tracking

The system 100 may be configured to carry out various algorithms in order to track hands and instruments that are present in video data generated by the video source(s) 200. By tracking the hands of the users along with the instruments disposed within the operating room, the system 100 can determine how the instrument suite 150 should be configured based on which of the instruments 154 are being used and/or which users are using the instruments 154. In this section, multiple implementations of algorithms for hand tracking, primary hand detection, bounding region generation, and instrument-in-hand detection are described in reference to Figure 4-7E.

A significant portion of the video analysis described herein is applied to the sub-region of the video data, rather than the entirety of the video data. Limiting the video analysis in this way can reduce computation cost and improve accuracy, and different methods of identifying the sub-region of the video data may be utilized depending on the implementation. Further, in some implementations, the sub-region is at least initially defined based on the hand of the user and may be referred to as a hand region.

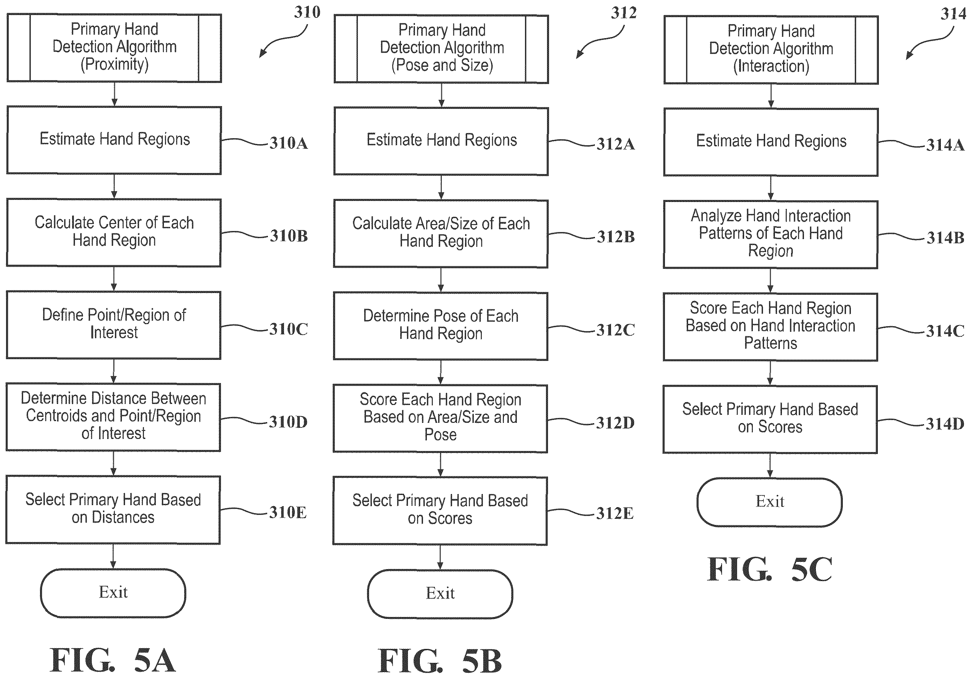

Referring to Figure 4, a hand detection algorithm 300 is illustrated according to one implementation. Starting at 300A, at least one feature, such as a set of landmarks, associated with the hand is detected. The features/landmarks may include hand features like palm centers, finger positions, finger joint positions, hand contours, or other features associated with hands. At 300B, a hand region of the video data is estimated based on the landmark(s) associated with the hand of the user. For example, the hand region may be a rectangular area of the video data which includes all of the detected hand features. In another example, a range of motion of the hand may be estimated based on the detected features, and the hand region may be defined so as to include all of the hand features throughout the range of motion of the hand. In some implementations, steps 300A and/or 300B may involve the detection of hand features of each hand of the user, including a primary hand and a secondary hand of the user, as further described below in reference to Figures 5A-5E. Additionally or alternatively, steps 300A and/or 300B may be carried out such that the hand region is estimated according to a bounding region algorithm described below. Regardless of implementation, at 300C, hand tracking is initialized in accordance with the detected hand features and the estimated hand region, and the estimated hand region may include the detected primary hand and/or the detected bounding region. Further, the hand region may be updated as the hand of the user moves within or relative to the hand region.

The hand detection algorithm 300 may also include a number of optional steps which are shown in Figure 4 as steps 300D through 300G. At 300D, the video data may be preprocessed. Various image/video preprocessing techniques are contemplated depending on the application of the algorithm 300. Video sources in the operating room often face dynamic lighting conditions, such as changes in intensity or color temperature. As such, techniques such as automatic white balancing (AWB) may be used to adjust the color balance and make white objects appear white by compensating for lighting variations. If the scene captured by the video source has a broad range of colors present, a gray world assumption technique may be used to perform white balancing of the video data and to remove color casts caused by lighting. Further, to ensure the fidelity of colors captured by the video source, a color correction matrix (CCM) may be used to compensate for inaccuracies in the video source's color representation by applying transformation matrices to map the captured color values closer to human vision or standardized colors. These color/white balancing techniques help ensure accurate color representation of objects captured by the video source, such and the patient, the user, and the instrument(s).

The preprocessing may involve illumination techniques meant to ensure a uniform level of brightness and minimize the effect of shadows and glare from lighting and reflective surgical instruments. For example, homomorphic filtering may be used to enhance image contrast by separating illumination and reflectance components of the image/video. Homomorphic filtering is particularly useful in compensating for uneven lighting in the operating room to ensure consistent illumination across the field of view of the video source. As another example, shadow removal techniques may be used to remove shadows cast by surgical instruments or the users of the surgical system 100.

The preprocessing may include contrast enhancement techniques for increasing visibility of important details within the video data, such as surgical incisions, tissues, or instruments, especially in low-light areas or when using endoscopes or laparoscopes. These techniques may include contrast limited adaptive histogram equalization (CLAHE) and/or gamma correction. CLAHE is used to improve the local contract in the video data by enhancing areas that are too dark or too bright without introducing noise. This is ideal for enhancing fine details that could otherwise be missed during surgery. Gamma correction, on the other hand, helps to brighten or darken images in a non-linear way, which allows enhanced visibility of objects and surfaces under uneven lighting conditions that are typical in surgery.

The preprocessing may include noise reduction techniques for enhancing clarity of the video data. For example, gaussian and median filtering may be applied to remove noise caused by variable lighting or slight camera movement. Gaussian and median filtering smooths the image by reducing the high-frequency noise. Median filtering is most effective in maintaining edges and preventing image blurring. As another example, temporal noise reduction techniques may be used to suppress noise and ensure smoother, clearer video without the loss of critical details.

The preprocessing may include edge detection and highlighting techniques to ensure that the edges of critical areas, such as hands of the user and surgical instruments, are clearly visible. This can help reduce the likelihood of errors during procedures. Some examples of edge detection and highlighting techniques include Canny edge detection, Sobel edge detection, and contour detection. Canny and Sobel edge detection are useful for identifying the edges of objects in line of sight of the video source and making these edges clearer. In the operating room, this can be crucial for highlighting the boundaries of users (*e.g*., hands thereof), tissues of the patient, and surgical instruments. Contour detection, in a similar vein, can help highlight and outline structures of interest to help users and the system 100 focus on key areas, such as hands and instruments.

The preprocessing may include image stabilization techniques to provide a stable and clear video feed even when the video source is in motion. These techniques may include motion estimation and correction methods and/or rolling shutter correction methods. In the operating room, even slight movements of the video source or the patient can cause blurring or disorientation in the video feed. Image stabilization (*i.e.,* motion estimation and correction) techniques like optical flow or Kalman filtering help compensate for such motion, resulting in smooth, stable video. At the same time, rolling shutter artifacts can occur when the video source moves while capturing an image, distorting the image. Techniques to correct for this, such as de-warping or model-based compensation, are critical for maintaining clear visuals.

The preprocessing may include real-time frame rate and temporal smoothing techniques to reduce jitter or lag in video playback or video data being analyzed. For example, frame rate interpolation and/or temporal smoothing may be used. First, if the video data frame rate fluctuates or is too low, interpolation techniques can be used to generate intermediate frames, ensuring a smooth video stream. Techniques such as optical flow help estimate motion between frames and create new ones. And second, by averaging or blending frames over time, temporal smoothing reduces flicker and noise caused by sudden light changes or quick movements of surgical instruments.

The preprocessing may include lens distortion correction to ensure that spatial dimensions and proportions in the video data accurately represent the real world. Some examples of these techniques include radial and tangential distortion correction and fish eye correction. For distortion correction, camera lenses, particularly those used for wide-angle or endoscopic applications, can introduce barrel or pincushion distortion, where straight lines appear curved. Correcting this distortion is crucial for maintaining accurate proportions in the field of view. As for fish eye correction, endoscopic and laparoscopic cameras often capture wide-angle views to cover more of the surgical area, but this can introduce a fish-eye effect. Fish-eye correction algorithms rectify this distortion, giving a more natural perspective.

The preprocessing may include background subtraction to isolate the surgical field from distractions, such as non-relevant areas or staff movements not relevant to the methods described herein. In some cases, foreground segmentation is used. It is often crucial to isolate the region of interest, such as surgical instruments, users, or tissues of the patient, from the background. Foreground segmentation techniques like thresholding, background subtraction, or region-based methods can help in separating these elements. In the same or other cases, dynamic background removal is used because the background can constantly change due to personnel movement or light reflections. Dynamic background subtraction helps focus the analysis or visualization solely on the surgical area.

The preprocessing may include compression techniques and optimization techniques for real-time transmission of the video data to the control system 199. For example, lossless compression may be used where the quality of the video data is essential. As another example, real-time compression may be used so long as acceptable quality is maintained. Other generic image/video preprocessing techniques may be used.

At 300E, the hand features may be updated. For example, since the video data includes a plurality of image frames captured at different times, the hand may move between image frames and the features detected at 300A may move as a result. As such, step 300E may include updating the position of the detected hand features. At the same time, the hand may change poses between image frames and new features may be detected. If so, the new features may be detected. The hand features may also change based on the preprocessing step 300D, and the change may be reflected when updating the hand features at 300E. If the hand features were updated, such as at 300E, the algorithm 300 may adjust the hand region at 300F. Like at 300B, the hand region may be estimated (i.e. adjusted) to include the updated hand features. Finally, at 300G, the hand of the user is tracked within the adjusted hand region. Any combination of steps 300D through 300G may be included in the hand detection algorithm 300.

The hand detection algorithm 300 has been described generally with respect to which hand of the user is detected and tracked. In some implementations, however, a primary hand of the user may be specifically detected and tracked, rather than any hand of the user. This is because the user is more likely to be performing important tasks, such as holding and/or using the surgical instrument 154, with their primary (i.e. dominant) hand. Thus, the hand detection algorithm 300 be configured to focus on tracking the primary hand of the user in accordance with one of the primary hand detection algorithms described below and shown in Figures 5A-5E.

Referring to Figures 5A-5E, multiple primary hand detection algorithms are shown. The primary hand detection (PHD) algorithms include: a first PHD algorithm 310 focused on determining the primary hand based on which hand of the user is within a proximity of a point/region of interest, such as a surgical site, a table on which surgical instruments 154 are placed, or other points/regions of interest; a second PHD algorithm 312 that determines the primary hand based on sizes and/or poses of the hands; a third PHD algorithm 314 which determines the primary hand based on hand interaction patterns; a fourth PHD algorithm 316 that relies on user selection to determine the primary hand; and a fifth PHD algorithm 318 configured to compare the detected hands against a database of hands to identify the primary hand. One or more of the PHD algorithms 310, 312, 314, 316, 318 may be implemented when detecting the hand of the user during any of the methods described herein, such as at steps 300A and 300B of the hand detection algorithm 300.

As shown in Figure 5A, the first PHD algorithm 310 starts with estimating hand regions at 310A. The hand regions may be estimated based on detected hand features like step 300B of the hand detection algorithm 300. That said, step 310A may include grouping the hand features into a first hand region, a second hand region, etc., according to the relation of the detected hand features. For example, a first palm center and a second palm center may be located in the video data. At the same time, a first set of finger landmarks may be detected near the first palm center while a second set of finger landmarks may be detected near the second palm center. In this example, the first hand region may be estimated as an area surrounding the first palm center and the first set of finger landmarks, and the second hand region may be estimated as an area surrounding the second palm center and the second set of finger landmarks.

After step 310A, the hand regions are related to a point/region of interest. To this end, at 310B, a center of each hand region is calculated. The centroids may be calculated based on the area/volume of each hand region, the hand features included in each hand region, or one of the hand features of each hand region (*e.g.,* the palm center). Once the center of each hand region has been calculated, the reference point/region of interest is defined at 310C. The point/region of interest may be defined based on the surgery being performed, a specific stage/step of the surgery being performed, an object detected in the video data, a center of the video data, a patient present in the video data, the location of at least one user, or a combination thereof. Subsequently, at 310D, determines a distance between each center and the point/region of interest. At 310E, the primary hand is selected based on the distances determined at 310D. Since it is often expected for the primary hand of the user to be near the point/region of interest, the hand region that is closer (*i.e.,* the to the point/region of interest may be selected as the primary hand region. For example, a first distance may be calculated according to the distance between the center of a first hand region, and a second distance may be calculated according to the distance between the center of a second hand region. If the first distance is less than the second distance, the first hand region may be selected as the primary hand region. Alternatively, if the second distance is less than the first distance, the second hand region may be selected as the primary hand region.

Further, as shown in Figure 5B, the second PHD algorithm 312 begins by estimating the hand regions at 312A. The hand regions may be estimated at 312A similar to how the regions are estimated at step 310A of the first PHD algorithm 310. Once the hand regions have been estimated, the second PHD algorithm 312 focuses on the area and pose of each hand region (or the pose of the hand within each hand region) to determine which hand region include the primary hand of the user. Thus, the area and/or size of each hand region is calculated at step 312B, and the pose of each hand/hand region is determined at 312C. Each hand region is then assigned a score according to its area/size and pose at 312D. Finally, at 312E, the hand region with the highest score is selected as the primary hand region.

Even further, as shown in Figure 5C, the third PHD algorithm 314 also begins by estimating the hand regions at 314A. Again, the hand regions may be estimated at 314A similar to how the regions are estimated at step 310A of the first PHD algorithm 310. Afterwards, at 314B, a hand interaction pattern is determined and analyzed for each hand region. Each of the hand regions estimated at 314A are then assigned a score based on the associated hand interaction pattern at 314C. Finally, the hand region with the highest score is selected as the primary hand region at 314D.

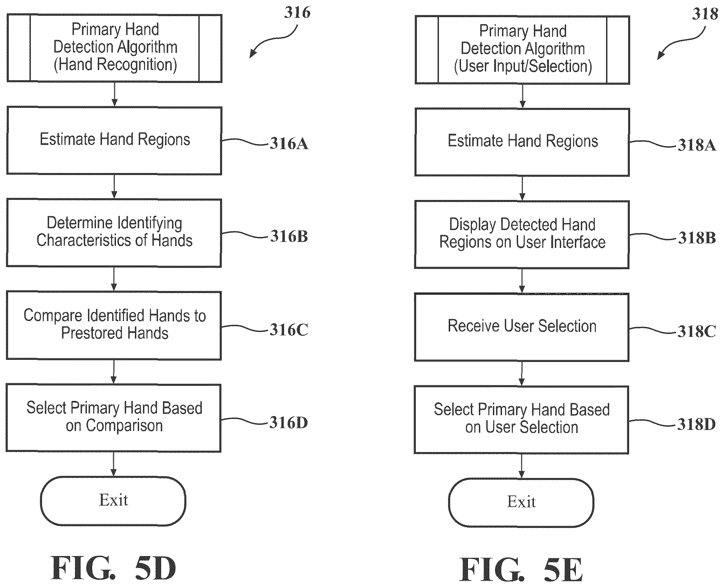

Yet further, as shown in Figure 5D, the fourth PHD algorithm 316 starts with estimating the hand regions at 316A. Like the previous algorithms 312, 314, the hand regions may be estimated in ways similar to step 310A of the first PHD algorithm 310. At 316B, identifying characteristics of each hand, such as vein patterns, hand shape, and/or biometric features are determined and associated with the appropriate hand region. Then, the identifying characteristics are compared to sets of hand characteristics stored in a database at 316C, and the primary hand is identified based on the comparison at 316D. For example, a specific user may have a unique visible hand vein that no other users have. If this hand vein appears in the hand region of the video data, the fourth PHD algorithm 316 may determine that this hand region corresponds to the primary hand of the specific user. Further, this comparison may be extensive so as to also differentiate primary hands from secondary hands, as well as hands of a first user versus hands of a second user.

Additionally, a user input or user identification may guide the primary hand detection of the fourth PHD algorithm 316. For example, the user input, such as a selection on a UI or a voice command, may be provided before step 316C and indicate that the primary hand is a right hand. Then, during the algorithm 316, the comparison and selection steps 316C, 316D may be limited to right hands. As another example, the user may be identified prior to step 316C and according to various methods discussed herein. The primary hand (*i.e.,* right or left hand) of the identified user may then be retrieved from a database, and the comparison and selection steps 316C, 316D may be limited to the whichever hand is the primary hand of the identified user. In either case, limiting the analysis in these ways can decrease computation cost and increase the efficiency of the algorithm 316.

Finally, as shown in Figure 5E, the fifth PHD algorithm 318 also starts with estimating the hand regions at 318A similar to step 310A of the first PHD algorithm 310, just like the other PHD algorithms 312, 314, 316. Unlike the other PHD algorithms 310, 312, 314, 316, the fifth PHD algorithm 318 includes a manual selection of the primary hand by the user. As such, at 318B, the hand regions are shown on a user interface. For example, the hand regions may be shown as bounding regions or bounding regions overlaid on the video data. At 318C, a user selection corresponding to a selection of a hand region is received. This selection allows the user to select which hand region include the primary hand manually. In response, the primary hand is set based on the user selection at 318D.

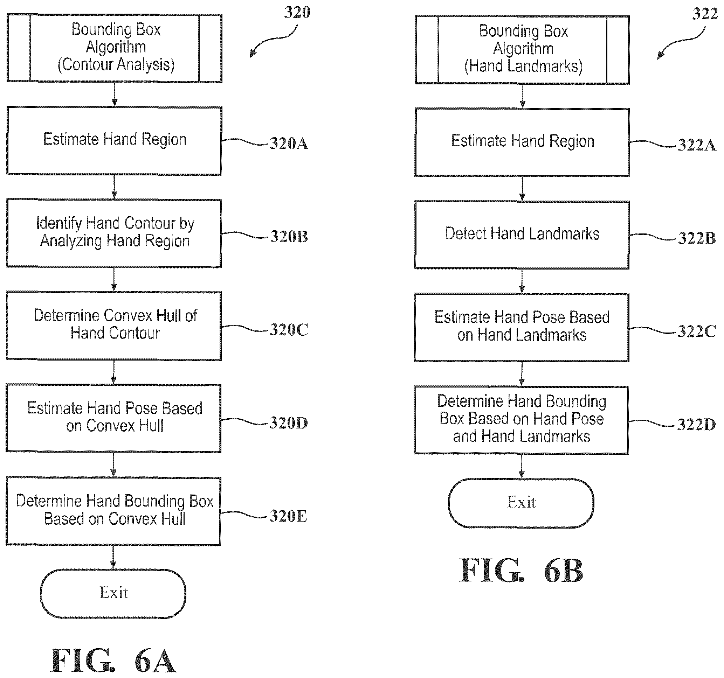

As described above, the sub-region of the video data may be identified based on the hand of the user, such as at step 300B of the hand detection algorithm 300 shown in Figure 4. Further, the system 100 may differentiate between the primary and second hands of the user or users in the event that multiple hands/hand regions are identified in the video data, like according to the primary hand detection algorithms 310, 312, 314, 316, 318 illustrated in Figures 5A-5E. Additionally, the sub-region may be identified as a bounding region surrounding the pertinent portion of the video data, and a series of bounding region algorithms configured to determine the bounding region are shown in Figures 6A-6D and described below. The bounding regions described herein can assume any suitable geometry, such as a square (box), rectangle, circle, or any shape specifically contoured to an object (like the hand or an instrument).

Referring to Figure 6A, a first bounding region algorithm 320 which is based on hand contour analysis is shown. Starting at 320A, the hand region is estimated. The hand region may be estimated similar to step 300B of the hand detection algorithm 300, and/or may be estimated according to one of the PHD algorithms 310, 312, 314, 316, 318. In any case, the algorithm 320 then moves to 320B. At 320B, a contour of the hand is identified by analyzing the hand region estimated at 320A. Based on the identified hand contour, a convex hull of the hand contour may be determined at 320C, and a hand pose may be estimated based on the convex hull at 320D. Finally, at 320E, the bounding region may be defined based on the convex hull.

Referring to Figure 6B, a second bounding region algorithm 322 that is based on hand landmark analysis is shown. Like the first bounding region algorithm 320, the second bounding region algorithm 322 begins at step 322A by estimating the hand region similar to step 300B of the hand detection algorithm 300 and/or one of the PHD algorithms 310, 312, 314, 316, 318. The algorithm 322 then proceeds to step 322B. At 322B, hand landmarks are detected. Using these hand landmarks, the pose of the hand is estimated at 322C. Finally, at 322D, the bounding region is determined based on the hand pose and/or the hand landmarks.

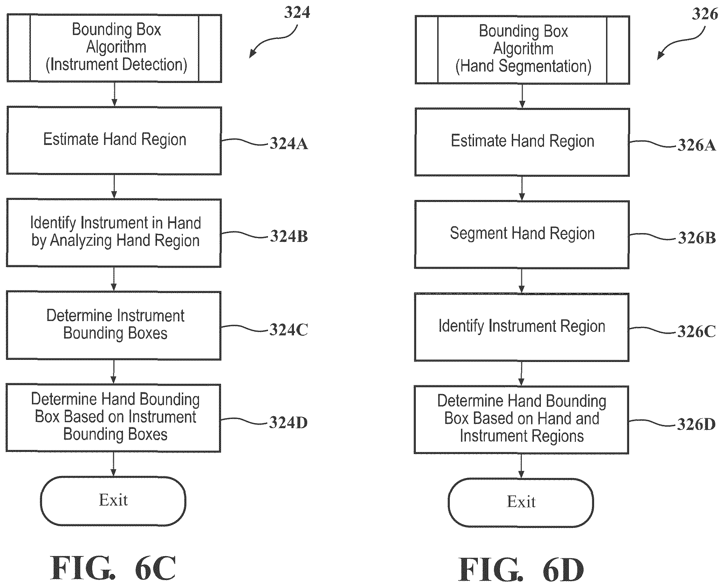

Referring to Figure 6C, a third bounding region algorithm 324 which is based on instrument detection is shown. Like the first/seconds bounding region algorithms 320, 322, the third bounding region algorithm 324 begins at step 324A by estimating the hand region similar to step 300B of the hand detection algorithm 300 and/or one of the PHD algorithms 310, 312, 314, 316, 318. The algorithm 324 then proceeds to step 324B. At 324B, any instrument(s) 154 being held in the hand of the user is identified. In some implementations, the instrument(s) 154 may be identified according to an object detection technique. In other implementations, the identity of the instrument(s) 154 may be assumed based on an operation or stage thereof being performed and optionally confirmed via an object detection technique. For example, if the user is following a surgical plan that is directing them to drill a screw into a bone of the patient, the instrument 154 may be assumed to be a drilling instrument. As another optional step, the control system 199 may trigger an alert (e.g., a popup notification on one of the displays 116, 117, 118) if the identity of the instrument 154 does not match the instrument associated with the surgical plan or step thereof. Subsequently, instrument bounding region(s) are determined at 324C such that the instrument bounding region(s) surround the instrument(s) 154 identified at 324B. Finally, at 324D, the bounding region is determined based on the instrument bounding region(s). More specifically, the bounding region may be determined so as to surround each of the instrument bounding regions. This may be useful where the instrument 154 is larger than the hand of the user.

Referring to Figure 6D, a fourth bounding region algorithm 326 that includes instrument detection and hand segmentation is shown. Like the other bounding region algorithms 320, 322, 324, the fourth bounding region algorithm 326 begins at step 326A by estimating the hand region similar to step 300B of the hand detection algorithm 300 and/or one of the PHD algorithms 310, 312, 314, 316, 318. The algorithm 326 then proceeds to step 326B. At 326B, the hand region is segmented by applying at least one segmentation algorithm to the hand region of the video data. For example, a binary segmentation algorithm may be applied to the hand region to identify which pixels/voxels of the hand region correspond to (1) the hand of the user and (2) not the hand of the user. In another example, a more advanced segmentation algorithm may be applied to the hand region which classifies the pixels/voxels into (1) the hand of the user, (2) the instrument 154, and (3) neither the hand nor the instrument 154. In any case, the algorithm 326 then proceeds to 326C. At 326C, an instrument region is identified based on the segmented hand region. Finally, at 326D, the bounding region is determined according to the hand and instrument regions. For example, the bounding region may be determined so that it surrounds the hand and instrument regions.

As described above, each of the bounding region algorithms 320, 322, 324, 326 include the determination of the bounding region. In some implementations, the bounding region may be determined so as to include a margin area surrounding the hand and/or the identified surgical instrument. The margin area may be based on hand size, instrument size, how much of the instrument is in within the bounding region, a combination thereof, or other suitable parameters. Additionally or alternatively, specific techniques of expanding the bounding region may be used.

A number of specific techniques of expanding the bounding region (*i.e.,* calculating the size of margin area) are contemplated. First, simple geometric expansion techniques may be used to expand the bounding region. For example, a fixed padding (in pixels or percentage) may be added to the bounding region. This can help include nearby regions of interest without relying on addition detection models. In some implementations, the fixed padding is set to 20% of the height/width of the bounding region. Second, an aspect ratio adjustment may be applied to the bounding region to ensure balanced framing around the hand of the user and the instrument being held by the user. For example, bounding regions with larger widths may be preferred where instruments tend to be longer than the hand of the user when held, while bounding regions with larger heights may be preferred where instruments tend to be taller than the hand of the user when held. Third and similar to the aspect ratio adjustment, a symmetry expansion technique may be used to extend the bounding region along the longer dimension of the bounding region. This can be useful when the user is using an instrument which extends along the axis of the hand when held. Fourth, the bounding region may be extended based on an output of an edge detection technique. For example, edges of the hand/instrument present in the bounding region may be identified. Once identified, the edges can be analyzed to find clusters of edge pixels near the hand, and the bounding region can be extended based on regions with dense edge pixel activity. Fifth, points/regions of interest related to the hand(s) of the user and/or the instrument 154 may be detected and used to extend the bounding region. For example, the bounding region may be extended so as to encompass fingertips of the hand, a proximal end of the instrument 154, and a distal end of the instrument 154. Sixth, the bounding region may be extending by using a distance-based graph traversal algorithm. More specifically, a spatial graphic may be created, where each node represents a part of the detected object (*e.g.,* the hand of the user) and the surrounding regions. With the nodes, a graph may be created where the nodes represent pixels/regions around the hand. Then, the bounding region may be extended using a connectivity or proximity threshold. In such a case, the bounding region may be extended to the point where adjacent regions no longer meet the connectivity/proximity threshold or no longer match the detected object. Seventh, the bounding region may be extending according to movement patterns of the tracked object (*e.g*., the hand). In order to do so, the control system 199 may calculate the movement direction to predict where the object will move, and then extend the bounding region in that direction. Eighth, density-based clustering algorithms may be used to expand the bounding region. For example, algorithms like DBSCAN may be used to identify clusters of pixels or regions that are likely to belong to the hand or instrument, and the bounding region can be extended to include the clusters. Any of these methods of expanding the bounding region/calculating the margin area may be used alone, in combination, or in part.

Further, while determining the bounding region, or once the bounding region has been determined, the algorithms 320, 322, 324, 326 may further include removing or disregarding portions of the video data which are outside of the bounding region. For example, the video data may be cropped around the bounding region. Even further, the bounding region algorithms 320, 322, 324, 326 have been described as estimating one hand region and defining a single bounding region around the hand region. That said, it is further contemplated to detect more than one hand in the video data, such as both hands of the user, and define bounding regions around each of the detect hands.

Even further, although the bounding region, the sub-regions, the hand regions, and the instrument regions are primary described herein as being determined relative to video data during singular steps of the methods and algorithms, each may be determined repeatedly during the methods/algorithms for each image frame of the video data. To this end, various methods of updating the bounding region and related regions may be employed. For ease of description, these techniques are described in reference to the bounding region but should be understood as being equally applicable to updating the sub-region, the hand region, and/or the instrument region.

In some implementations, the bounding region is updated according to movement prediction algorithms and methods. In a first case, a Kalman filter may be applied to the video data to estimate the state, such as the position and/or velocity, of the object being tracked (*e.g.,* the hand of the user). For example, the filter may be initialized based on a position of the bounding region in a first image frame. The position of the bounding region in a second image frame may then be predicted based on the state of the objects relative to which the bounding region was defined, like the hand of the user. This type of tracking is most advantageous where the bounding region is defined relative to objects that move in predictable manners, and the prediction can even handle short-term occlusion of the object from the video source. In a second case, an optical flow method, such as the Lucas-Kanade Optical Flow method, may be used to calculate the updated position of the bounding region. The optical flow methods calculate object motion between consecutive image frames by analyzing the apparent motion of pixels. As an example, the control system 199 may compute a motion vector field that describes the movement of image points between a first image frame and a second image frame. Based on the motion vector field, the expected motion of the bounding region may be estimated. In a third case, non-parametric models, like Mean-Shift or CamShift, are used to track and update the position of the bounding region based on a color map of objects within the bounding region. As the color map moves, the bounding region may be updated accordingly.

In some implementations, deep learning algorithms may be used to update the bounding region from a first image frame to a second image frame. In one case, a Siamese network, such as SiamRPN or SiamMask, may be used to learn the similarity between an object in a first image frame and the object in subsequent image frames. A Siamese network may be trained with two image frames, where the bounding region is identified in each image frame, and the network may then determine further bounding region locations based on matching the appearance of related objects in a frame subsequent to the two frames used to train the network. These networks are robust to variations in object appearance and occlusions and can also track at real-time speeds. In another case, a correlation filter, such as a Kernelized Correlation Filters (KCF) or a Discriminative Correlation Filter with Channel and Spatial Reliability (CSRT), may be used to track the bounding region between frames. Correlation filters are used to extract object features from an initial image frame, and then track the object by maximizing the correlation between the object features and subsequent image frames. Correlation filters are fast and efficient for real-time tracking, especially for high-resolution video streams. In yet another case, a DeepSORT algorithm may be used to track the bounding region. The DeepSORT algorithms incorporates appearance features from a deep learning model for more robust tracking in the presence of multiple similar objects (*e.g*., multiple hands). In order to do so, the detection of the object related to the bounding region is initialized using a CNN-based detector, like YOLO or Faster R-CNN, and a combination of Kalman filtering and appearance descriptors are used to continuously track the object, and therefore the bounding region.

In some implementations, hybrid tracking methods may be used to update the bounding region. In a first case, a track by detection method may be carried out by detecting the object in a first image frame, and then using a tracking algorithm to estimate the position of the object in a second image frame without re-detecting the object. For example, a deep learning model, such as YOLO or Faster R-CNN, may be used to detect the object periodically, while a traditional tracking algorithm like Kalman Filter or Mean-Shift tracks the detected object between detection. Hybrid tracking methods are robust to appearance changes and occlusion and reduce the computation cost of detecting in every frame. In a second case, an object re-identification (Re-ID) algorithm may be used to track the bounding region. This technique combines object tracking with re-identification when the object is temporarily lost due to occlusion, frame gaps, or other causes. As an example, deep learning may be used to extract a feature vector (*i.e.,* appearance descriptor) related to the object. If the object becomes occluded or leaves the field of view of the video source(s), the object is re-identified based on a similarity of the feature vector to previously stored descriptors when it reappears.

Any of the methods of updating the bounding region and related regions may be used alone, in combination, or in part. The same methods may also be used, in whole or in part, in combination with one of the methods/algorithms described in the sections below.

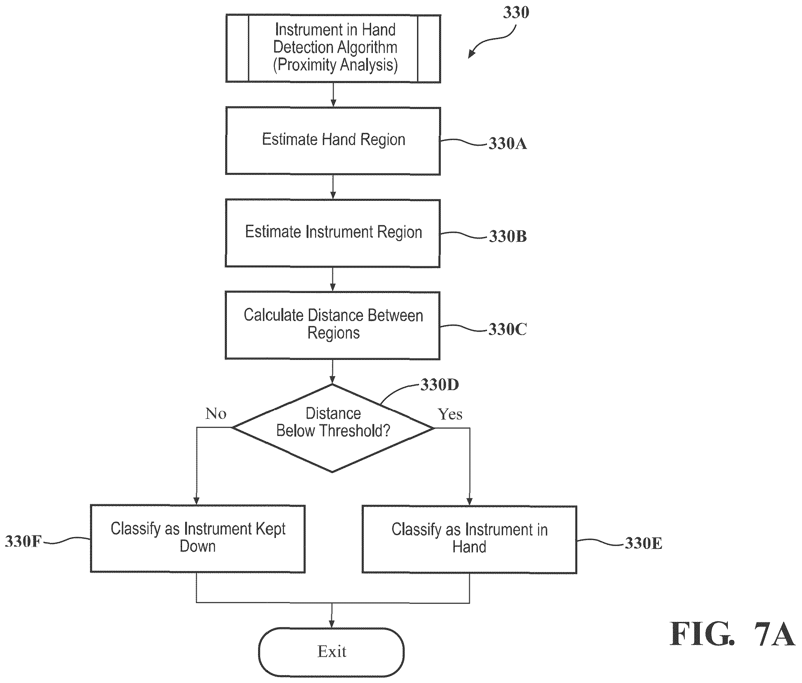

The hand tracking, primary hand detection, and bounding region algorithms are important steps for many of the implementations of the surgical system/instrument 100, 154 control methods described herein. After a combination of these algorithms, however, some implementations of the control methods further include steps/algorithms for determining whether the surgical instrument 154, or which instrument 154 of the instrument suite 150, is being used by the user. To this end, various implementations of an instrument-in-hand detection (IIHD) algorithms are shown in Figures 7A-7E.

Referring to Figure 7A, a first IIHD algorithm 330 is shown. The first IIHD algorithm 330 focuses on detecting a proximity between the hand of the user and the instrument(s) 154 to determine if an instrument is being used. Starting at 330A, the hand region is estimated, such as according to one of the various implementations of hand region estimation described above. Once the hand region is estimated, an instrument region is estimated at 330B. Then, at 330C, a distance between the hand region and the instrument region is calculated. At 330D, the distance between the two regions is compared against a threshold distance to determine if the two regions are within a threshold distance of one another. If so, the algorithm 330 moves to 330E and classifies the hand region or bounding region as corresponding to "instrument-in-hand." If the hand and instrument regions are not determined to be within the threshold distance of one another at 330D, however, the hand region or bounding region may be classified as "instrument-kept-down."

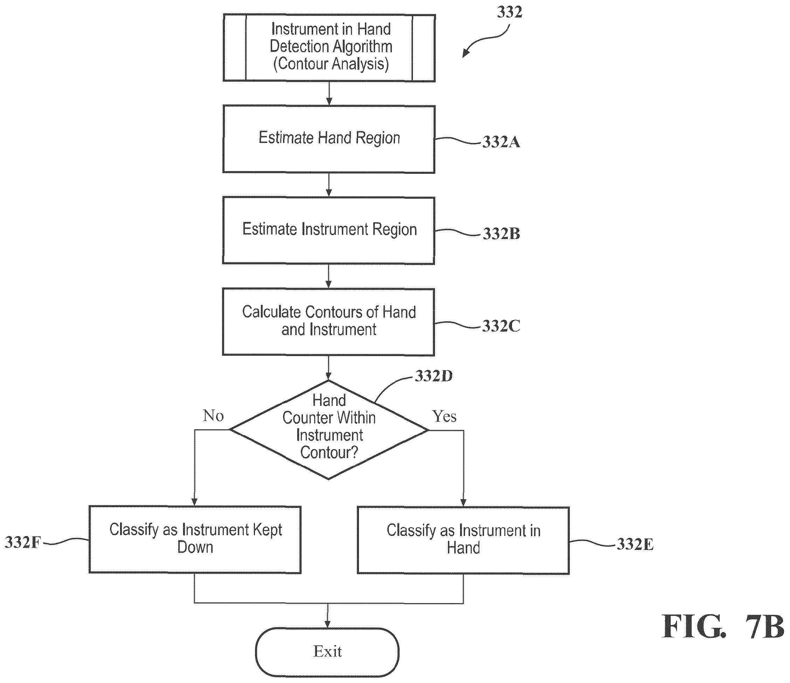

Referring to Figure 7B, a second IIHD algorithm 332 is shown. The second IIDH algorithm 332 relies on hand and instrument contour analysis to determine if an instrument is being used. The second algorithm 332 starts off effectively the same as the first IIHD algorithm 330, with the hand region and the instrument region being estimated at 332A and 332B, respectively. At 332C, the second IIHD algorithm 332 diverges from the first algorithm 330, and the contours of the hand and the instrument 154 are analyzed. For example, the algorithm 332 may apply Sobel/Canny edge detection techniques to analyze the contours of the hand and/or the instrument 154. Subsequently, at 332D and based on the analysis performed during 332C, the algorithm 332 determines if the hand contour is within the instrument contour. If so, the algorithm 332 proceeds to 332E and classifies the hand region or bounding region as corresponding to "instrument-in-hand." If the hand contour is determined to be outside of the instrument contour at 330D, however, the hand region or bounding region may be classified as "instrument-kept-down."

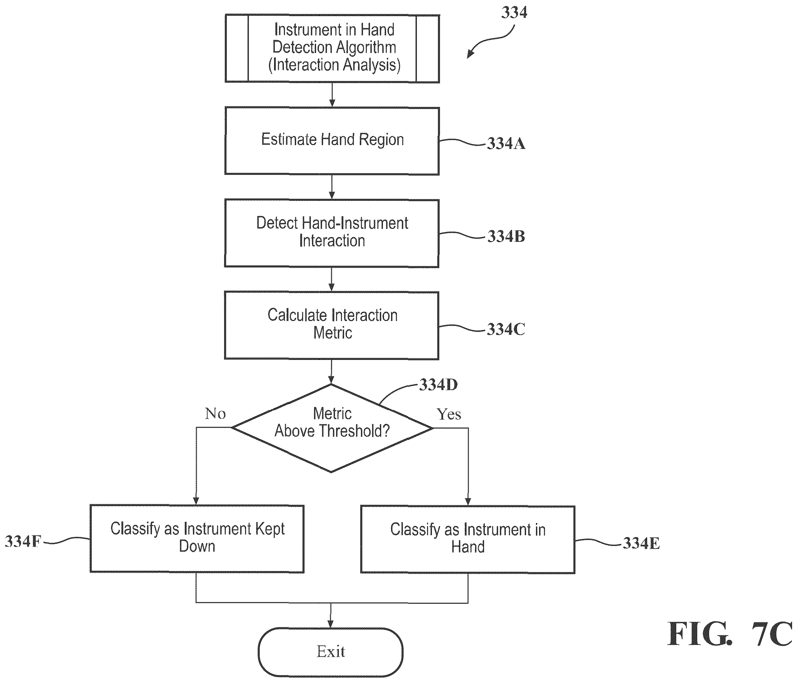

Referring to Figure 7C, a third IIHD algorithm 334 is shown. While the first and second algorithms 330, 332 rely on proximity and contour analysis, the third IIHD algorithm 334 performs more complex interaction analysis to determine if an instrument is being used. Beginning at 334A, the hand region is estimated, such as according to one of the various implementations of hand region estimation described above. Afterwards, at 334B, interactions between the hand of the user and the instrument 154 are detected. An interaction metric is then calculated based on the detected interactions at 334C. For example, the interaction metric may be calculated based on criteria for determining significant hand-instrument interactions. Then, at 334, the interaction metric is compared against a threshold, such as an interaction score threshold. If the interaction metric is above the threshold, the algorithm proceeds to 334E and the hand region and/or the bounding region is classified as "instrument-in-hand." If the interaction metric is below the threshold, the algorithm 334 instead moves to 334F and the hand region and/or the bounding region is classified as "instrument-kept-down."

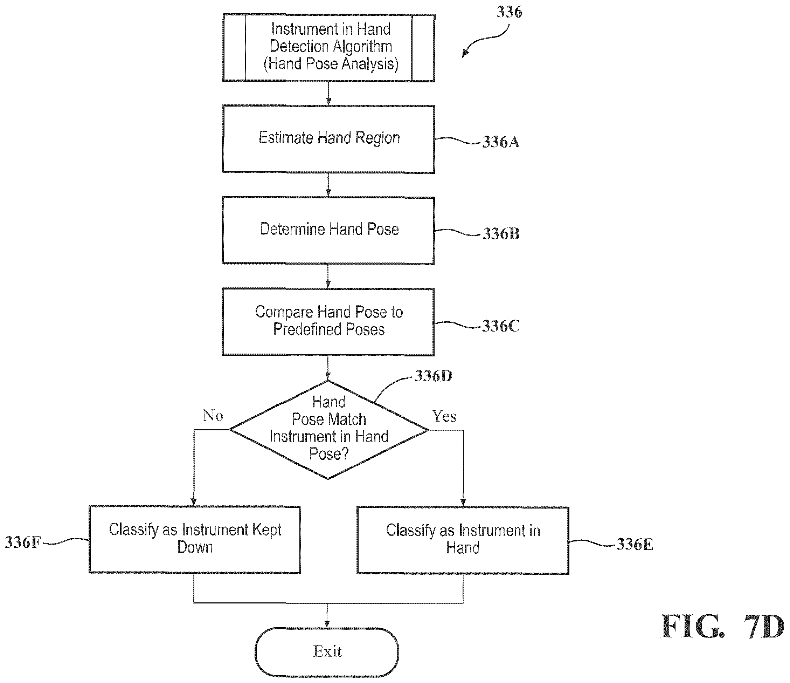

Referring to Figure 7D, a fourth IIHD algorithm 336 is shown. Unlike the others, the fourth IIHD algorithm 336 focuses entirely on the hand of the user, rather than analyzing a relationship between the hand and an instrument. Starting at 336A, the hand region is estimated, such as according to one of the various implementations of hand region estimation described above. After the hand region has been estimated, the pose of the hand within the hand region is determined at 336B. At 336C, the pose of the hand is compared to predefined hand poses. Based on the comparison, the algorithm 336 determines if the hand pose matches a predefined pose that is associated with an instrument being held in the hand at 336D. If so, the algorithm 336 continues to step 336E, at which point the hand region is classified as "instrument-in-hand." If the hand pose does not match a pose associated with holding an instrument, the algorithm 336 moves to step 336F and classifies the hand region as "instrument-kept-down."

In another implementation of the fourth IIHD algorithm 336, the user may be identified prior to step 336C, such as according to a user input or biometric features of the hand extracted from the video data during step 336A. Then, at step 336C, the hand pose may be compared against predefined hand poses which are associated with the identified user and optionally stored in a database. Limiting the comparison in this way may help to decrease the computational cost thereof. Additionally, the user may handle instruments differently than another user, and focusing the comparison on only predefined hand poses associated with the identified user may increase the accuracy of the algorithm by decreasing the chances of a false match.

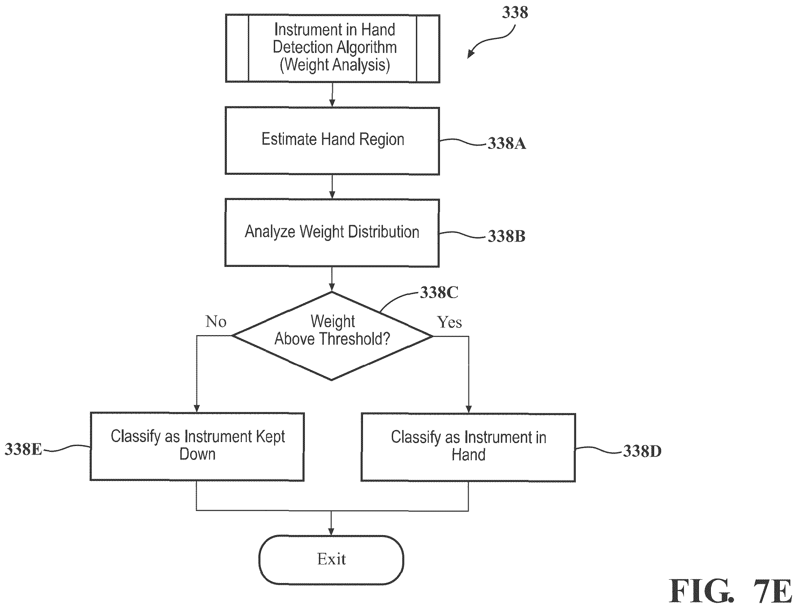

Referring to Figure 7E, a fifth IIHD algorithm 338 is shown. Similar to the fourth algorithm 336, the fifth IIHD algorithm 338 relies only on the hand of the user to determine if an instrument is being used. Differently here, however, is that the fifth algorithm 338 uses weight distribution data from sensors attached to the user to determine if an instrument is being used. Starting at 338A, the hand region is estimated, such as according to one of the various implementations of hand region estimation described above. At 338B, the algorithm 338 analyzes the weight distribution data associated with the hand region using pressure-sensitive techniques or wearable sensors. Afterwards, at 338C, the weight distribution data is compared against a weight distribution threshold. If the weight distribution data is above the weight distribution threshold, the algorithm 338 continues to 338D and classifies the hand region as "instrument-in-hand." If not, the algorithm 338 proceeds to 338E and classifies the hand region as "instrument-kept-down."

The IIHD algorithms 330, 332, 334, 336, 338 described above each include the step of classifying the hand region as either "instrument-in-hand" or "instrument-kept-down." That said, in implementations where the sub-region of the video data is determined as including the hand region and the instrument region, the sub-region of the video data, rather than the hand region, may be classified. Further, the sub-region may be classified based on the classification of the hand region.

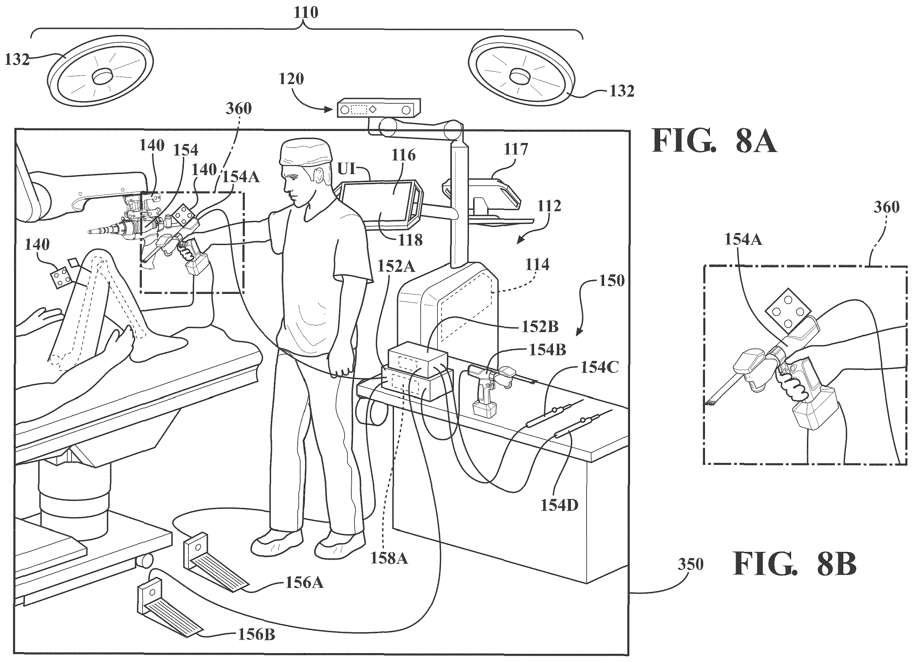

Referring to Figures 8A-11B, an example of hand and instrument tracking is shown. The example shown in these figures is meant to be illustrative of a generic application of the algorithms described in this section, as well as the methods described in the sections below. At a high level, each of Figures 8A, 9A, 10A, and 11A show the navigation system 110 along with an example of a field of view 350 of the navigation system 110 which is representative of the video data generated by at least one video source of the navigation system 110. The field of view 350 is representative of the video data generated by the navigation system 110, and it will be understood that a reference to the field of view 350 is analogous to a reference to the video data. To exemplify identifying a sub-region 360 of the video data that include the hand of the user, tracking the hand in the sub-region 360, and detecting a presence of surgical instrument within the sub-region 360, Figures 8B, 9B, 10B, and 11B illustrate the various positions and contents of the sub-region 360.

Starting with Figures 8A and 8B, the user is shown holding the first surgical instrument 154A near the patient. At this point, the sub-region 360 includes the first instrument 154A, which is connected the first surgical console 152A. Since the first instrument 154A is present in the sub-region 360, the control system 199 may detect the presence of the instrument 154A and cause the first console 152A to activate and/or set the operational parameters of the instrument 154A. At the same time, because the other instruments 154B, 154C, 154D are not present in the sub-region 360, the control system 199 may deactivate these instruments 154, 154C, 154D.

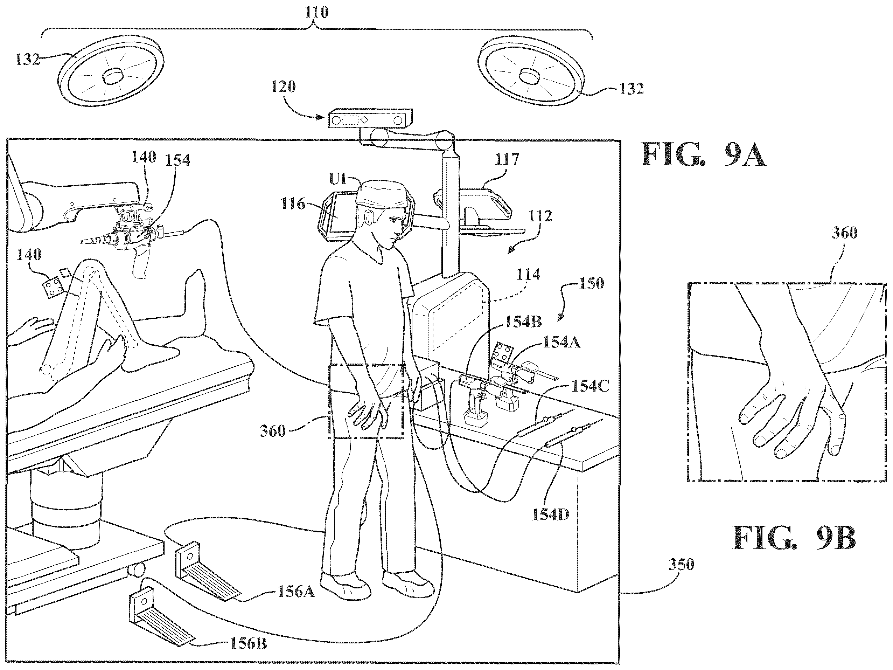

Now referring to Figures 9A and 9B, the user has finished using the first surgical instrument 154 and is shown as having placed the first surgical instrument 154A down with the rest of the instrument suite 150. Although not shown, the sub-region 360 may have been periodically updated as the user moved their hand. In any case, since none of the instruments 154A, 154B, 154C, 154D are present in the sub-region 360, the control system 199 may deactivate all of the instruments 154, 154B, 154C, 154D.

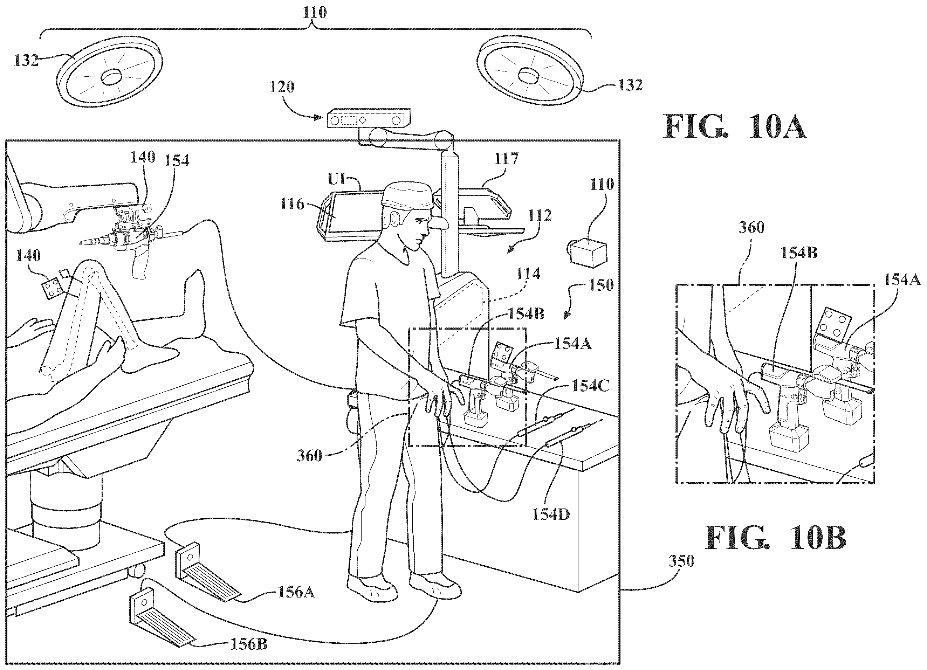

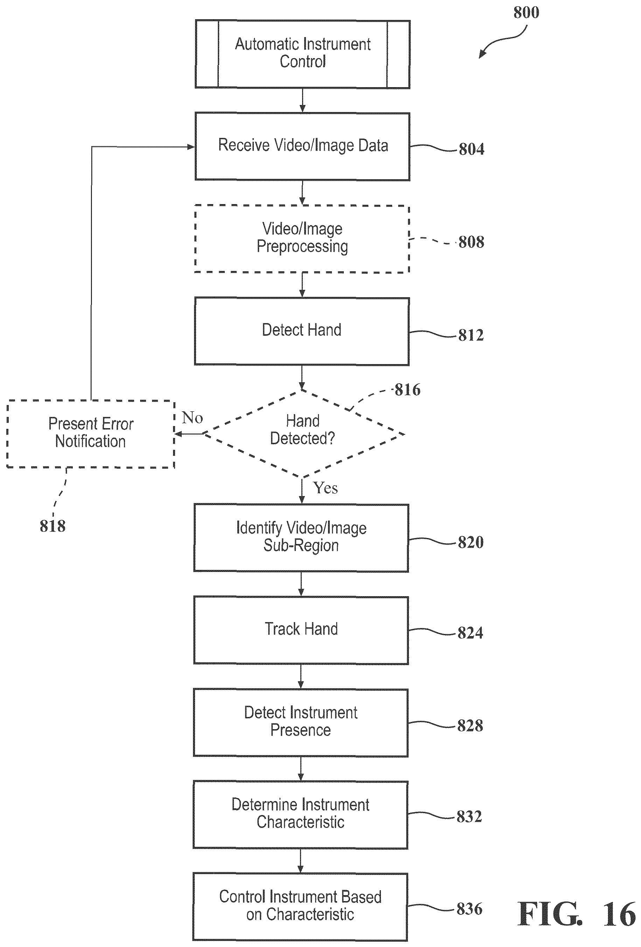

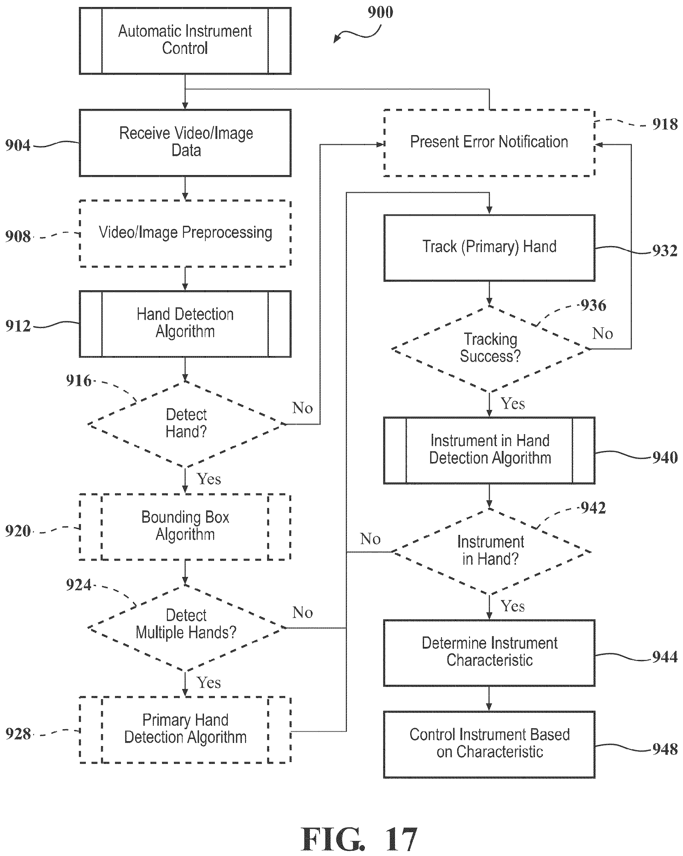

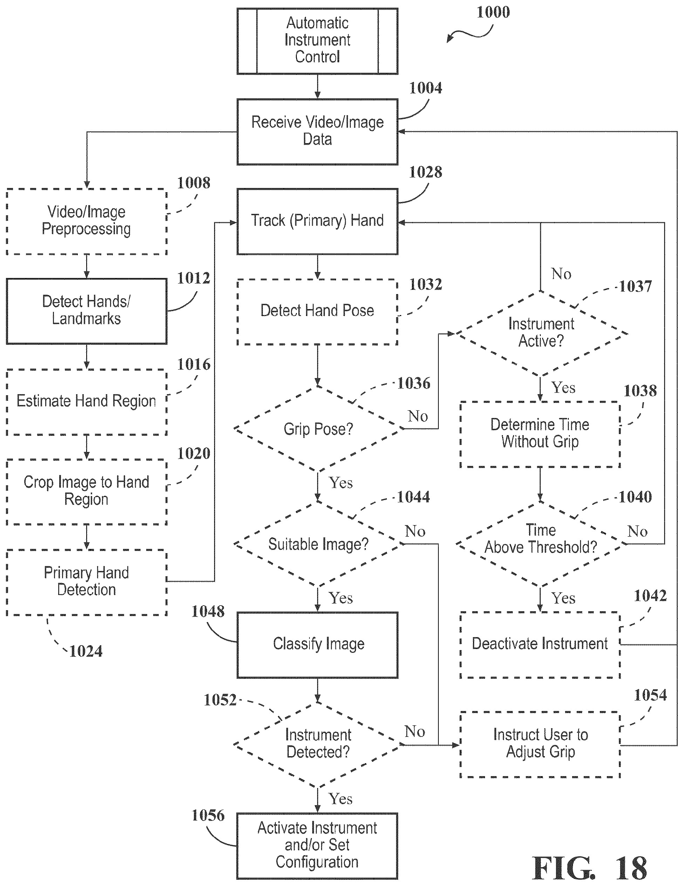

Moving to Figures 10A and 10B, the user is shown with their hand near, but not grabbing, the second surgical instrument 154B. As such, the control system 199 may determine that the second surgical instrument 154B is present in the sub-region 360. At this point, however, the control system 199 may control the instrument suite 150 depending on the implementation and/or method being carried out. For example, the control system 199 may activate and/or set the operational parameters of the second surgical instrument 154B based on presence within the sub-region 360 alone (*e.g.,* see Figure 16). In another example, the control system 199 may employ one of the IIHD algorithms 330, 332, 334, 336, 338 and classify the sub-region 360 as instrument-kept-down (*e.g*., see Figure 17). In this example, the second surgical instrument 154B may be left deactivated in response to the instrument-kept-down classification. As yet another example, the control system 199 may determine if the pose of the hand matches a grip pose in order to determine if the instrument 154B should be activated and/or operational parameters set (*e.g.,* see Figure 18).

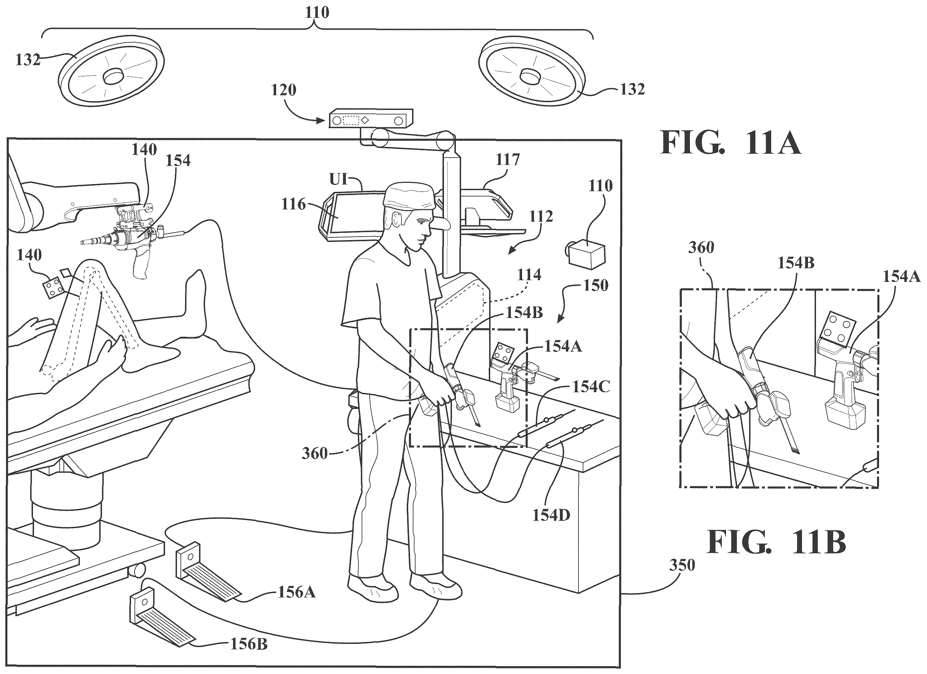

Finally, referring to Figures 11A and 11B, the user is shown gripping and moving the second surgical instrument 154B away from the rest of the instrument suite 150. Since the second instrument 154B is present in the sub-region 360 and the user is gripping the second instrument 154B, the control system 199 may activate and/or set the operational parameters of the second instrument 154B based on at least one of the presence of the instrument 154B, an output of one of the IIHD algorithms 330, 332, 334, 336, 338, the grip pose of the hand of the user, and a user identification. As described below, there may be situations in which the hand of the user occludes the instrument being picked up. To avoid issues causes by this occlusion, it is further contemplated for the system to retrieve the sub-region 360 of a previous frame of the video data, and attempt to detect the presence of (*i.e.,* identify) the instrument being picked up. In the illustrated implementation, the second instrument 154B is partially occluded in the sub-region 360 shown in Figure 11B but otherwise fully visible in the sub-region 360 shown in Figure 10B. As such, the control system 199 may determine that an instrument is being picked up based on the sub-region 360 of Figure 11B, and then identify the instrument as the second instrument 154B based on the sub-region 360 of Figure 10B.

As an outcome of the example of tracking and sub-region analysis illustrated in Figures 8A-11B, the control system 199 may automatically deactivate the first surgical instrument 154A, activate the second surgical instrument 154B, and/or change the operational parameters of the first surgical console 152A (or the instrument 154B if not connected to a console). Further, although the sub-region is described generally with respect to Figures 8A-11B, it is contemplated that any of the algorithms described in this section, or methods described in other sections, may be used to identify the sub-region 360 of the field of view/video data 350.

### III. Methods for Multiple Camera Tracking

Each of the algorithms described in the previous section may be carried out by only one of the localizer 120 and the boom cameras 132. That said, the algorithms may be performed with data from more than one video source 200. For example, the hand detection and primary hand detection algorithms 300, 310, 312, 314, 316, 318 may be carried out using video data from the localizer 120, both of the boom cameras 132, and the HMD. Using multiple video sources can help the system 100 increase the field of view of the navigation system 110 and reduce situations where an instrument and/or a hand becomes occluded from view of the navigation system 110. More specifically, using multiple video sources adds spatial and angular diversity, which can resolve issues of occlusion or object loss in one video source by leveraging data from other video sources.

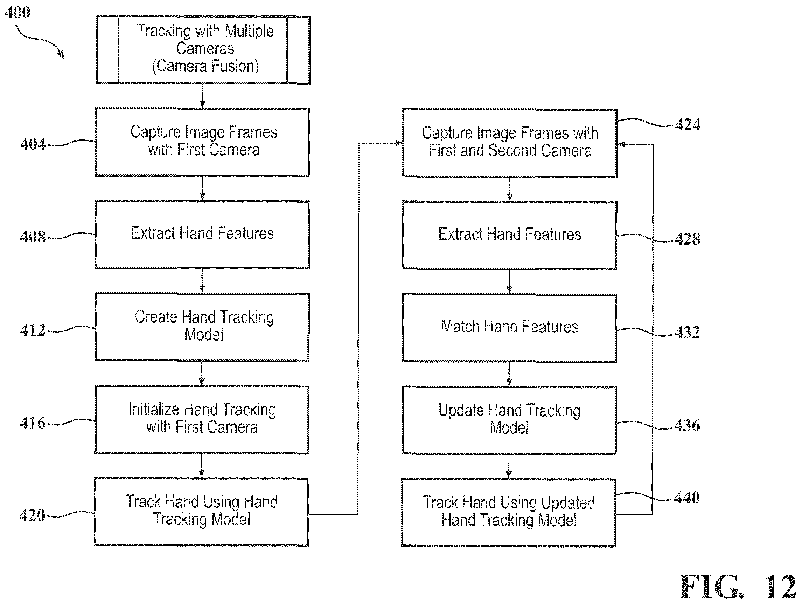

Referring to Figure 12, a first multiple camera tracking method 400 is shown. The first multiple camera tracking method 400 is based on a fusion of video data from multiple video sources. Starting at 404, a first video source captures video data. At 408, a first set of hand features are extracted from the video data. After the first set of hand features are extracted, a hand tracking model is created based on the first set of hand features at 412. Then, at 416, hand tracking is initialized using the hand tracking model and the first video source. At 420, the hand is tracked by the first video source in accordance with the hand tracking model. The method 400 may proceed to step 424 once a second video source detects the hand of the user, after the second video source is initialized, or whenever it is otherwise advantageous to begin tracking with both video sources. In any case, the method 400 proceeds to step 424 and causes both the first and second video sources, such as the boom cameras 132, to capture video data. If more than two video sources are available, the step 424 may include capturing video data with the three or more video sources, such as the localizer 120 and the two boom cameras 132.

After the multiple video sources capture video data at 424, the method 400 includes updating the hand tracking model according to steps 428 through 436. Starting at 428, a second set of hand features are extracted from the video data created by the second video source. If additional video source are available, additional sets of hand features may be extracted at 428 as well. After extracting the hand features from the video data created by the second video source, the second set of hand features (and additional sets of hand features, if extracted at 428) are matched to the first set of hand features at 432. Then, at 436, the hand tracking model is updated based on the matching performed at 432.

The matching and updating steps 432, 436 are useful for determining whether the hand features extracted from the first and second video sources correspond to the same hand or to different hands. In one example, the hand tracking model created at 412 using the first set of hand features may correspond to the primary hand of a surgeon, while second and third sets of hand features may be extracted from the second video source and correspond to the primary hand of the surgeon and a primary hand of a nurse assisting the surgeon, respectively. By matching the second and third sets of hand features to the first set of hand features, the system 100 may determine that the third set of hand features are not associated with the primary hand of the surgeon, while the second set of hand features are associated with the primary hand of the surgeon. Using this information, the hand tracking model may be updated based on only the first and second sets of hand features. This way, the hand tracking model is not erroneously updated based on a combination of hands which are different and potentially related to different users.

Finally, at 440, the hand of the user is tracking based on the updated hand tracking model and using the first and second video sources. As shown in Figure 12, the method 400 may return to step 424 while/after tracking at 440 in order to further update the hand tracking model. This may be in response to an additional video source becoming available, a determination that the first or second video sources on longer detect the hand while the third video source can detect the hand, or another scenario in which further updating the hand tracking model is desired.

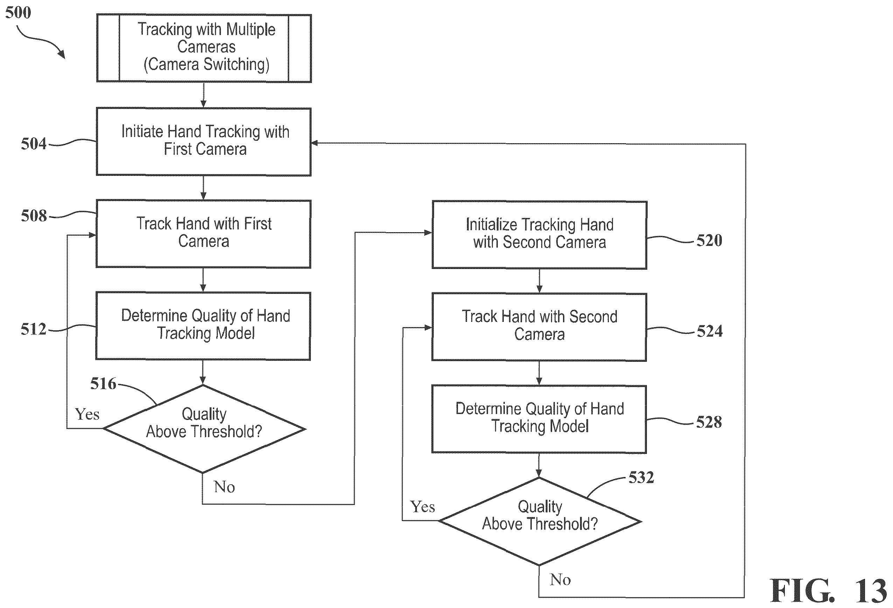

Referring to Figure 13, a second multiple camera tracking method 500 is shown. Instead of combining video data from multiple video sources like the first method 400, the second multiple camera tracking method 500 involves switching between the video sources based on tracking data quality. Starting at 504, hand tracking with the first video source is initialized. Although not shown in the figures, step 504 may include, or be prefaced by, steps similar to steps 404 through 416 of the first multiple camera tracking method 400. More specifically, at 504, a set hand features may be extracted from the video data generated by the first video source, and a first hand tracking model may be created based on the first set of hand features. In any case, the method 500 moves to step 508 after initializing hand tracking at 504. At 508, the hand is tracked with the first video source using the hand tracking model. While tracking the hand with the first video source, the method 500 loops between steps 508 and 516. The loop includes determining a quality of the first hand tracking model at 512 and comparing the quality to a threshold quality at 516. If the quality of the first hand tracking model is above the threshold quality, the method 500 loops back to 508. If the quality is below the threshold, however, the method 500 moves to step 520 to switch the video source from the first video source to the second video source.

At 520, tracking using the second video source is initialized. Similar to step 504, tracking with the second video source may be initialized by extracting a second set of hand features from the video data generated by the second video source, and a second hand tracking model may be created based on the second set of hand features. In some implementations, the second hand tracking model may be created based on a combination of the first set of hand features, the second set of hand features, and the first hand tracking model. For example, probabilistic data association or graph-based matching may be used to combine the sets of hand features to create the second hand tracking model. At 524, the hand is tracked with the second video source using the second hand tracking model. Another loop then occurs between steps 524 and 532. Like the previous loop from step 508 to step 516, the loop includes determining a quality of the second hand tracking model at 528 and comparing the quality to the threshold quality at 532. If the quality of the second hand tracking model is above the threshold quality, the method 500 loops back to 524. If the quality is below the threshold, however, the method 500 moves to step 504 and reinitialized tracking with the first video source.

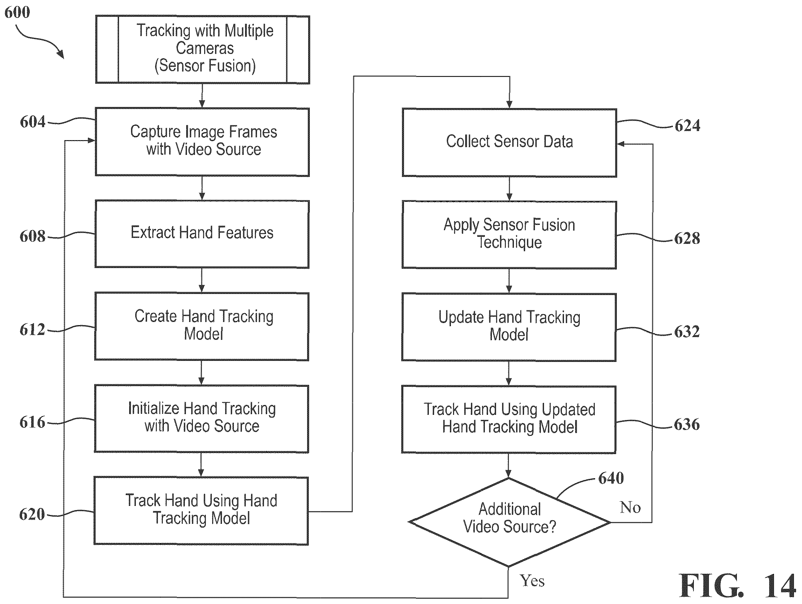

Referring to Figure 14, a third multiple camera tracking method 600 is shown. Unlike the first two methods 400, 500, the third multiple camera tracking method 600 further relies on sensor data, such as from sensors coupled to the hand of the user, in combination with the video data to tracking the hand of the user. Starting at 604, the video data is generated by a first video source. The video data is then analyzed, and hand features are extracted at 608. Subsequently, at 612, a hand tracking model is created based on the extracted hand features. At 616, hand tracking with the first video source is initialized. After which, the hand of the user is tracked by the first video source and in accordance with the hand tracking model at 620. After step 620, sensor fusion begins at 624. At 624, sensor data generated by wearable sensors worn on or near the hand(s) of the user is collected. The wearable sensors may include inertial measurement units (IMUs), passive trackers, active trackers, near-field communication (NFC) enabled devices, RFID devices, Bluetooth devices, and the like. In one implementation, the wearable sensors are radiopaque gloves worn by the user to increase the visibility of the hand to the video source 200. In another implementation, the wearable sensors are IMUs that track the pose of the hand of the user relative to a sensor coordinate system. Once the sensor data has been collected, the method 600 proceeds to step 628. At 628, at least one data fusion technique, such as a sensor fusion algorithm or Kalman filter, is used to combine and integrate the sensor data with the hand tracking model. In other words, the sensor data and the image data may be transformed into a common coordinate system such that the hand is tracked relative to the common coordinate system by both the wearable sensor(s) and the video source(s). By using multiple tracking sources, such as the localizer 120 and the wearable sensor, the accuracy of the tracking may be increased. Further, the system 100 may be able to rely on one of the two tracking sources in a situation where tracking data from one source becomes unreliable, such as the hand becoming occluded from view of the localizer 120. Some examples of suitable data fusion techniques are described in U.S. Application Publication No. 2024/0041542, entitled "Techniques For Detecting Errors Or Loss Of Accuracy In A Surgical Robotic System," the entirety of which is incorporated by reference. Based on the output of the sensor fusion techniques, the hand tracking model is updated at 632. Then, at 636, the hand of the user is tracked using the updated hand tracking model.

While tracking the hand of the user with the first video source at 636, the method 600 proceeds to step 640 and loops back to one of steps 604 and 624 based on whether an additional video source is detected at step 640. If no additional video sources are detected, the method 600 loops back to step 624, and more sensor data is collected and used to further update the hand tracking model. In other words, the outputs of the wearable sensors are continuously monitored and used to calibrate the sensor fusion technique to ensure optimal performance. Once the method 600 reaches step 640 and a second video source is detected, either initially or after looping back to step 624 any number of times, the method 600 loops back to step 604. At this point, the method 600 repeats, except each step is performed based on video data from each of the video sources. For example, hand features can be extracted from video data from the first video source as well as the second video source and used to update the hand tracking model at 612 (rather than create the model like the first pass of the method 600). Continuing with the example, sensor data can again be collected at 624 and the sensor fusion techniques may be used to update the hand tracking model in accordance with the sensor data and the hand features extracted from the video data generated by the first/second video sources. The method 600 may loop any number of times depending on the number of video sources detected.

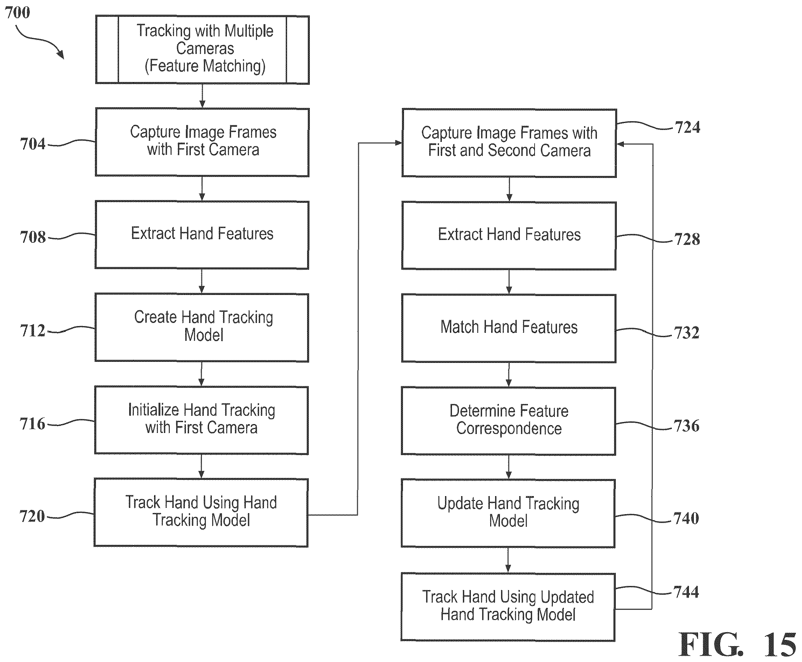

Referring to Figure 15, a fourth multiple camera tracking method 700 is shown. The fourth multiple camera tracking method 700 is based on extracting and matching features from video data generated by multiple video sources. Beginning at 704, a first video source captures video data. At 708, a first set of hand features are extracted from the video data. After the first set of hand features are extracted, a hand tracking model is created based on the first set of hand features at 712. Then, at 716, hand tracking is initialized using the hand tracking model and the first video source. At 720, the hand is tracked by the first video source in accordance with the hand tracking model. The method 700 may proceed to step 724 once a second video source detects the hand of the user, after the second video source is initialized, or whenever it is otherwise advantageous to begin tracking with both video sources. In any case, the method 700 proceeds to step 724 and causes both the first and second video sources, such as the boom cameras 132, to capture video data. If more than two video sources are available, the step 724 may include capturing video data with the three or more video sources, such as the localizer 120 and the two boom cameras 132.

After the multiple video sources capture video data, the method 700 includes updating the hand tracking model according to steps 728 through 740. Starting at 728, a second set of hand features are extracted from the video data created by the second video source. If additional video source are available, additional sets of hand features may be extracted at 728 as well. After extracting the hand features from the video data created by the second video source, the second set of hand features (and additional sets of hand features, if extracted at 728) are matched to the first set of hand features at 732. The matching step 732 may be carried out similarly to step 432 of the first multiple camera tracking method 400 illustrated in Figure 12.

At 736, a correspondence and/or an alignment is determined between (1) the hand features extracted from the video data generated by the first video source and (2) the hand features extracted from the video data generated by the second video source. More specifically, certain hand features from the first and second sets of hand features may be representative of the same part of the hand of the user, and combining the features which are related to the same part of the hand of the user may result in a more accurate understanding of this aspect of the hand. For example, the system 100 may identify which of the second set of hand features corresponds to features of the first set of hand features, such as the pointer finger of the primary hand of the surgeon. With the features so matched, the hand tracking model may be updated to include details of the matched hand features from multiple perspectives. For example, the updated model may be able to estimate an expected pose of the hand feature relative to the second video source based on a determined pose of the hand feature relative to the first video source. This can increase the efficiency of hand detection.

At 740, the hand tracking model is updated based on the matching performed at 732 and the correspondence/alignment determining at 736. Finally, at 744, the hand of the user is tracking based on the updated hand tracking model and using the first and second video sources. As shown in Figure 15, the method 700 may return to step 724 while/after tracking at 744 in order to further update the hand tracking model. This may be in response to an additional video source becoming available, a determination that the first or second video sources on longer detect the hand while the third video source can detect the hand, or another scenario in which further updating the hand tracking model is desired.

In some implementations of the methods 400, 500, 600, 700, the first video source may be different in modality or resolution compared to the second video source, and the first video source may be used to make initial tracking determinations to decrease the computation cost incurred by the second video source when tracking the hand of the user. For example, the first video source may be a thermal camera while the second video source may utilize machine vision. In this example, the thermal camera may be used to estimate the sub-region of the video data generated by the machine vision camera, after which the second video source may determine the sub-region of the video data that includes the hand of the user based on the initial estimate generated using the first video source. This way, the computation cost incurred by the second video source is decreased. At the same time, the accuracy of hand detection may be increased where the modality of the first video source is especially suited to the detection of the human body, like a thermal camera, while the second video source is better suited for instrument detection.

In some implementations of the methods 400, 500, 600, 700, the first video source may be used to track users within the operating room, and the second video source may be used to track surgical instruments and/or other objects. Further, a transform/registration between the first and second video sources may be known to the control system 199, and the control system 199 may employ the first video source for tracking the hand(s) of each user and the second video source for determining which instrument was picked up by the user(s). For example, the control system 199 may track the hand and wait for an instrument to be present near the hand, such as within a sub-region of the video data generated by the first video source as described herein. Once an instrument is detected near the hand (e.g., according to one of the IIHD algorithms 330, 332, 334, 336, 338), the control system 199 may employ the second video source to determine which instrument is near or was picked up by the hand being tracked with the first video source. This may include determining the location of a sub-region of the second video source based on the sub-region of the video data captured by the first video source as well as the registration/transform mentioned above. With the sub-region so located, the control system 199 may identify the instrument as the instrument present in the sub-region of the video data captured by the second video source. The control system 199 may even reference the sub-regions of the video data from both video sources to increase the accuracy of instrument detection. For example, the sub-region associated with the first video source may be analyzed to identify the instrument, and the control system 199 may then compare the instrument identified in this sub-region with the instrument identified in the sub-region associated with the second video source. If the instruments match, the control system 199 may determine characteristics of the instrument and control the instrument based on the characteristics like described below. If the instruments do not match, however, the control system 199 may trigger an alert or instruct the user to present the instrument to one of the video sources so that the instrument may be reidentified. Additionally or alternatively, the control system 199 may determine a confidence associated with the identification of the instrument based on the sub-region of the video data captured by the first video source, and/or determine a confidence associated with the identification of the instrument based on the sub-region of the video data captured by the second video source. If the confidence associated with the first video source is high enough (e.g., above a threshold), the control system 199 may rely solely on the first video source for instrument identification. If the confidence associated with the first video source is low (e.g., below the threshold), the control system 199 may rely solely on the second video source for instrument identification. And if the confidence associated with both video sources, the control system 199 may trigger an alert, instruct the user to present the instrument to one of the video sources so that the instrument may be better identified, or identification may be performed with a combination of the sub-regions associated with each video source.

In some implementations of the methods 400, 500, 600, 700, the first video source may be used to track a first user, the second video source may be used to track a second user, and the control system 199 may control specific instruments depending on which of the first and second users picked up and/or is holding the instrument. For example, the first user may be a nurse, and the second user may be a surgeon. In this example, the control system 199 may identify the instrument when it is picked up by the nurse and set the configuration of the control console associated with the instrument as described herein. At the same time, the control system 199 may keep the instrument deactivated until the instrument is identified as picked up by or in the hand of the surgeon. This way, the control console is ready to control the instrument according to the correct parameters, but inadvertent activation of the instrument is avoided prior to the surgeon grabbing/holding the instrument.

The multiple camera tracking methods 400, 500, 600, 700 may additionally include steps to ensure synchronization, calibration, and maintenance of consistent object identity across video sources. First, camera calibration may be used which involves estimating intrinsic (camera-specific) and extrinsic (position relative to each other) parameters, allowing seamless integration/synchronization of views from multiple perspectives. For example, a calibration technique which uses multiple images of a known calibration pattern (e.g., one of the trackers 140) to compute camera parameters, such as the Zhang's method, may be used to synchronize frames across video sources to ensure that temporal consistency is maintained. Second, in order to maintain consistent object identity across video sources, such as when updating the hand tracking model and tracking the hand using the update model at steps 436 and 440 of the first multiple camera tracking method 400, different methods may be employed. In a first example, the system 100 may estimate the 3D position of an object (*e.g*., the hand) by analyzing images from two or more video sources which have overlapping fields of view. More specifically, the object may be identified in the video data received from two different video sources, and triangulation may be used to compute the pose of the object. Then, the object may be tracked by combining object detection and pose estimation. This can provide more robust tracking in complex environments, like when occlusions occur often. In a second example, epipolar geometry may be leveraged to establish correspondences between the views of the object as captured by the different video sources. More specifically, after detecting the object in a first video source, an epipolar line may be computed in a second video source. Then, the system 100 may search along the epipolar line to identify the corresponding position of the object in the video data associated with the second video source. In a third example, where the video sources share overlapping fields of view but 3D tracking is not necessary, homography-based methods can transform the position of the object from the 2D plane of a first video source to a 2D plane of a second video source. This is a simple and effective method for tracking in 2D space across multiple view without requiring full 3D reconstruction. In a fourth example, Re-ID (described above) may be used, and optionally combined with Kalman Filtering, for tracking across large spaces and/or where the video sources do not have overlapping fields of view. More specifically, the feature identification/matching steps may be used to reidentify the hand if the hand becomes occluded or leaves the field of view of any one video source. Any of these additional steps be used alone, in combination, or in part.

### IV. Automatic Console Configuration

The system 100 may be configured to automatically configure elements of the instrument suite 150 according to various methods. Additionally, the system 100 may leverage the algorithms described in Section II and the methods described in Section III to automatically configure the instrument suite 150. To this end, various methods of controlling the system 100 are described in this section and in reference to Figures 16-18. The methods are described as being carried out by the control system 199 for clarity, but any or all of the methods may instead be executed by any part of the control system 199, such as the navigation controller 114 and/or the instrument controller 158. Further, some of the methods are described in reference to video data, but image data may be used instead.

Referring to Figure 16, a method 800 of automatically configuring and controlling at least one of the instruments 154 of the instrument suite 150 according to one implementation is illustrated. At 804, the control system 199 receives video data (or image data) depicting at least a portion of the operation room. The video data may be generated by the navigation system 110, such as by the localizer 120, the boom camera(s) 132, or both. Once the video data is received, the method 800 may proceed to a preprocessing step at 808. At 808, the video is preprocessed, such as according to one of the preprocessing techniques described above in reference to step 300D of the method 300. After step 804 or step 808, the method 800 proceeds to step 812.

At 812, the control system 199 detects a hand of the user within the video data. In some implementations, the method 800 includes a detection confirmation step at 816. At 816, the control system 199 optionally determines if the hand of the user was detected at 812. If not, the method proceeds to step 818. At 818, an error notification is presented to the user. The error notification may be presented on any or all of the displays 116, 119 and may include instructions to the user to adjust the position of the localizer 120 and/or the boom camera 132 to ensure that a line of sight between the device(s) 120, 132 and the hand of the user is not occluded. After step 812, and optionally step 816, the method proceeds to step 820.

At 820, the control system 199 identifies a sub-region of the video data that includes the hand of the user, for example, in accordance with one of the bounding region algorithms 320, 322, 324, 326. After identifying the sub-region of the video data, the hand of the user is tracked in the sub-region of the video data at 824. At 828, the control system 199 analyzes the sub-region of the video data in an attempt to detect a presence of the surgical instrument 154 in the sub-region. In some cases, the method 800 can optionally loop between steps 824 and 828 and continuously track the hand of the user until the presence of the instrument 154 is detected. After the presence of the surgical instrument 154 is detected, the method 800 continues to step 832. At 832, at least one characteristic of the surgical instrument 154 is determined in response to the presence of the surgical instrument 154 having been detected in the sub-region of the video data. The characteristic(s) of the instrument 154 may include any physical characteristic thereof, such as a shape, profile, color, or other physical property of the instrument 154. Additionally or alternatively, the characteristic(s) of the instrument 154 may be determined based on a pose of the hand holding the instrument 154 and detected at 812. For example, specific instruments may be held in such a way that the hand of the user forms a unique shape, or has uniquely spaced hand landmarks (e.g., finger locations). As such, the instrument 154 may be characterized according to the pose of the hand in addition to, or alternatively to, physical characteristics of the instrument 154 itself. Further, the user may be identified during the method 800. Then, the hand pose may be compared to predefined hand poses associated with the identified user. These predefined hand poses may be further correlated to specific instruments (i.e., how the hand is historically posed when holding each instrument) and the instrument 154 may be identified based on the comparison. Even further, an operation, or step/stage thereof, being performed may be determined via user input or other means during the method 800. This information may be combined with the identity of the user and compared against predefined hand poses that describe how the identified user holds specific instruments during the operation or step/stage of the operation. The operation and/or operation step/stage information may also be relied upon without the identity of the user, and the hand pose may be compared to predefined hand poses which describe how most users hold specific instrument during the operation or step/stage thereof. The characterization step 832 may also be carried out by inputting the sub-region of the video data into a model which was previously trained to recognize the instrument based on at least one of a physical characteristic of the instrument, an identity of the user, a hand pose, an operation being performed, and a step or stage of the operation being performed. After the at least one characteristic of the instrument 154 is determined, the instrument 154 is controlled based on the determined characteristic(s) at 836.

Referring to Figure 17, a method 900 of automatically configuring and controlling at least one of the instruments 154 of the instrument suite 150 based on a combination of the algorithms described in Section II is illustrated. Starting at 904, the control system 199 receives video data depicting at least a portion of the operating room. The video data may be like that received at step 804 of the previous method 800. Once the video data is received, the method 900 optionally includes a preprocessing step at 908, during which the video data may be preprocessed according to at least one preprocessing technique described above in reference to step 300D of the method 300.

After the video data is received and optionally preprocessed, a hand detection algorithm is applied to the video data at 912. For example, the hand tracking module 212 may receive the video data from the video source and apply the hand detection algorithm 300 shown in Figure 4. In some implementations, a hand region and/or sub-region including the hand of the user may be identified at 912. After applying the hand detection algorithm, the method 900 may proceed to step 916 and determine whether at least one hand was detected in the video data. If no hands were detected, the method 900 may move to step 918 and present an error notification to the user, such as by causing one of the displays 116, 117 of the navigation system 110 to show a pop-up notification. If at least one hand was detected at 916, however, the method 900 may continue to step 920.

At 920, a bounding region algorithm may be applied to the video data. For example, the hand tracking module 212 may apply one of the bounding region algorithms 320, 322, 324, 326 shown in Figures 6A-6D. After determining the bounding region(s) using the bounding region algorithm, the method 900 may continue to step 924 and determine whether more than one hand was detected based on the output of the hand detection algorithm applied at 912 or the output of the bounding region algorithm applied at 920. If not, the method 900 may proceed to step 932. If more than one hand was detected, the method 900 may apply a primary hand detection algorithm at 928. For example, the hand tracking module 212 may apply one of the PHD algorithms 310, 312, 314, 316, 318 shown in Figures 5A-5E. The method 900 may then move to step 932.

At 932, the hand of the user may be tracked within the sub-region of the video data. For example, the navigation system 110 may track the hand of the user and may use the hand tracking module 212 to do so. If the navigation system 110 includes more than one video source 200, like the localizer 120 and the two boom cameras 132 of the illustrated implementation, one of the multiple camera tracking methods 400, 500, 600, 700 shown in Figures 12-15 may be carried out during step 932. Subsequently, at 936, the method 900 may determine whether the tracking is successful. If not, the method 900 may jump back to step 918 and present an error notification. On the other hand, if hand tracking is successful, the method 900 may proceed to step 940.

At 940, the method 900 may include determining whether an instrument is present in the sub-region of the video data and/or whether the instrument is being used by the user. More specifically, one of the IIHD algorithms 330, 332, 334, 336, 338 shown in Figures 7A-7E may be executed by the instrument-in-hand detection module 218. Optionally, at 942, the method 900 may include determining whether an instrument is in the hand of the user. If not, the method 900 may loop back to step 932 upon a determination that the user is not using an instrument. On the other hand, if the user is holding an instrument, the method 900 then continues to step 944. At 944, the video data is analyzed in order to determine characteristics of the instrument held by the user. The characteristics of the instrument may be determined similar to step 832 of the previous method 800. For example, the control system may employ the instrument classification module 216 to characterize the instrument based on the physical appearance of the instrument. Finally, at 948, the instrument is controlled based on the characteristic(s) determined at 944.

Referring to Figure 18, a method 1000 of automatically configuring and controlling at least one of the instruments 154 of the instrument suite 150 according to another implementation is illustrated. Beginning at 1004, similar to the previously described method 800, 900, the control system 199 receives video data depicting at least a portion of the operation room. Although video data is received, the video data may be received one image frame at a time, and the entirety of the method 1000 is herein described as being carried out on the image frame. The method 1000 may then return to step 1004, a second image frame may be received by the control system 199, and the remainder of the method 1000 may be carried out on the second image frame (and so on). The video data may be generated by the navigation system 110, such as by the localizer 120, the boom camera(s) 132, or both. Once the image frame is received, the method 1000 may proceed to step 1008, at which point the image frame may be preprocessed, such as according to one of the preprocessing techniques described above in reference to step 300D of the method 300. After step 1004 or step 1008, the method 1000 proceeds to step 1012.

At 1012, The control system 199 detects a hand of the user within the image frame. Optionally, the control system may then estimate at least one sub-region of the image frame that includes the hand at 1016, and crop the image frame to the sub-region at 1020. If multiple hands are detected at 1012, step 1016 may include estimated a plurality of hand regions, and step 1020 may include cropping the image frame to each of the hand regions. In other words, the control system 199 may generate a cropped image for each hand region, each cropped image including at least one hand. This may be done by applying one of the bounding region algorithms 320, 322, 324, 326 to the image frame, and cropping the image frame to the bounding region. Assuming that multiple hands were detected and a plurality of cropped images were generated, the control system 199 may detect which of the sub-regions (*i.e.,* cropped images) correspond to the primary hand of the user at 1024. After step 1012 or one of the optional steps 1016, 1020, 1024, the method 1000 continues to step 1028.

At 1028, the (optionally, primary) hand of the user is tracked, such as by the hand tracking module 212. In implementations where individual image frames are being analyzed, the method 1000 may continuously loop between steps 1004 and 1028, during which the hand may be tracked by continuously (1) detecting hands within newly received image frames, (2) estimating hand regions for each image frame, (3) cropping each image frame to the respective hand regions, and (4) selecting the hand regions that includes the primary hand of the user for each image frame. Where only one hand/sub-region is estimated, and only one cropped image generated per image frame, primary hand detection may not be performed. In any case, the remaining steps of the method 1000 may be continuously performed for each image frame received at 1004. For example, a first loop of steps 1004 through 1028 may be executed, while a second loop of steps 1032 through 1056 are applied to each image frame received and analyzed during the loop of steps 1004 through 1028.

At 1032, the cropped image frame is analyzed to detect a pose of the hand. Subsequently, at 1036, the hand pose is compared to known hand poses to determine if the hand pose corresponds to a grip pose. For example, the control system 199 may feed the cropped image frame to the gesture recognition module 214, and the module 214 may compare the pose of the hand within the cropped image frame against a set of predefined hand poses that each correspond to the hand gripping an instrument. If the hand pose matches a grip pose, the method 1000 continues to step 1044. If not, the method 1000 moves to step 1037, at which point the control system 199 determines if an instrument that was previously controlled during the method 1000 is still activated. For example, if the method 1000 previously determined that the first instrument 154A was being used, the first instrument 154A would have been activated and the first console 152A may have been configured according to an appropriate set of operational parameters. In this scenario, at step 1037, the control system 199 may determine if the first instrument 154A is still active. If not, the method 1000 returns to step 1028. If the instrument is still active, the method 1000 proceeds to step 1038. At 1038, the control system 199 calculates an amount of time without a grip pose being detected. And at 1040, the amount of time is compared against a threshold time frame. In implementations where the video data includes a plurality of image frames, the amount of time may be determined according to a count of consecutive image frames without the grip pose, and the threshold time frame may be defined as a threshold count of consecutive image frames. If the amount of time is below the threshold time frame, the method 1000 returns to step 1028. If the amount of time without a grip pose is above the threshold time frame, however, the control system 199 may assume that the instrument is no longer being used, deactivate the instrument at step 1042, and then return to step 1028. If there were no instruments active upon reaching step 1037, the method 1000 returns to step 1028.

At 1044, the control system 199 may determine if the cropped image frame is suitable for the purposes of controlling the instrument 154 and/or configuring the instrument suite 150. The step 1044 may include determining if the hand of the user is likely to be occluding the instrument 154 present in the cropped image frame. Additionally or alternatively, the step 1044 may include comparing a confidence of the hand detection at 1012, and/or a confidence of the hand tracking at 1028, against a confidence threshold. If the confidence of hand detection/tracking is below the confidence threshold, the control system 199 may conclude that the image is not suitable at 1044. There are situations where the cropped image frame is suitable for detecting/tracking the hand but not the instrument 154, especially where the instrument 154 is relatively small, and this step 1044 helps to eliminate cropped image frames that are only suitable for part of the method 1000. For example, the control system 199 may detect a blurring of at least a portion of the cropped image frame. In many cases, the hand detection/tracking is more robust and capable of handling blurred hand regions, while instrument detection is more prone to error when blurring is involved. As another example, debris (*e.g*., blood) may cover the instrument 154, the hand, or even the video source where the video source is an HMD. If only the instrument 154 is affected by the debris in this example, the cropped image frame may be suitable for hand detection/tracking, but unsuitable for instrument detection. However, if the debris are present on the hand but not the instrument 154, the cropped image frame may be suitable for both hand and instrument detection even though the hand is partially covered in debris. Slightly differently, if the debris is present on the HMD, a portion of the video data may be partially obscured, and the cropped image frame may only be suitable if limited to the unobscured portion of the video data.

If the cropped image frame is found to be unsuitable at 1044, the method 1000 may jump to step 1054 and instruct the user to adjust their grip so as not to occlude the instrument 154 from the video source 200. The method 1000 may then return to step 1004. In some implementations, the control system 199 may utilize a first cropped image frame generated during a first iteration of the method 1000 for hand pose detection, but also generate a second cropped image frame during a second iteration of the method 1000 for instrument detection. For example, the first cropped image frame may correspond to a first image frame of the video data generated at a first time, while the second cropped image frame may correspond to a second image frame of the video data generated at a second time which is different form the first time. Once suitable cropped image(s) are generated, the method 1000 continues to step 1048. Conversely, if the cropped image frame is found to be suitable (*e.g*., for both hand detection and instrument detection) at the first iteration of step 1044, the method 1000 continues to step 1048.

At 1048, the control system 199 classifies the cropped image frame. For example, the control system 199 may apply one of the IIHD algorithms 330, 332, 334, 336, 338 to classify the cropped image frame according to whether the hand of the user is gripping/holding the instrument 154. Additionally or alternatively, the cropped image frame may be classified according to characteristics of the instrument, which may be determined similar to steps 944 and 832 of the methods 800, 900 illustrated in Figures 16 and 17. After classifying the cropped image frame, the control system 199 may determine whether the instrument 154 was detected while classifying the image frame at 1052. More specifically, the cropped image frame may be classified at step 1048 according to specific features of the instrument 154, such as the type of instrument and/or the end effector coupled to the instrument 154. Based on an analysis of the classified cropped image frame, if at least one of the type of instrument and type of end effect could not be detected, or if the instrument 154 was not detected at all, the method 1000 may go to step 1054 and instruct the user to adjust their grip like described above. If the instrument and its features were detected when classifying the cropped image frame, the method 1000 continues to step 1056. At 1056, the control system 199 activates the instrument 154 and/or sets the configuration for the console 152 controlling the instrument 154.

The final steps 836, 948, 1056 of each of the methods 800, 900, 1000 are directed to controlling an instrument and/or a console controlling the instrument. Although the steps 836, 948, 1056 are described generally, controlling the instrument/console may be carried out in more specific manners. For example, controlling the instrument/console 154, 150 may include automatically adjusting operational parameters of surgical instruments, automatically linking and unlinking external control devices and surgical instruments, and/or automatically enabling and disabling surgical instruments based on which instruments are picked up/set down, among other features. The operational parameters may include torque level, end effector RPM, braking/acceleration of end effectors, oscillation frequency, and irrigation rates/suction force, among others.

The control system 199 may have access to a database which includes a list of users and corresponding preferred operational parameters. This database may be the same database discussed above in reference to primary hands of users such that an identification of the primary hand of the user may also inform the system 199 of the preferred operation parameters. Further, the database may have multiple sets of preferred operational parameters, each set of preferred operation parameters relating to a specific instrument/instrument type, associated with each user. Even further, since some instruments may be configured to receive end effectors with differing shapes, sizes, functions, etc., the database may include multiple sets of preferred operational parameters associated with each user and relating to a combination of a specific instrument/instrument type and a specific end effector/end effector type. Thus, during the final steps 836, 948, 1056, the control system 199 may activate the detected instrument and set the operational parameters according to at least one of the preferences of the user, the detected instrument/instrument type, and the detected end effector/end effector type.

In some implementations of the methods 800, 900, 1000, the methods 800, 900, 1000 are run continuously as instruments are picked up, put down, moved around, handed between users, reconfigured, and coupled to different end effectors. This way, the system 100 can be reconfigured as the state of the operating room changes. In one example, there may be multiple users operating on the patient, like a surgeon performing an operation and a nurse assisting the surgeon. When the surgeon requires an instrument, the nurse may be the one to pick the instrument up and hand it to the surgeon. In this example, the control system 199 may be configured to receive an input from the nurse, such as a voice command, informing the system 199 that the user is not the person initially holding/picking up the instrument but is instead the surgeon. Alternatively, the control system 199 may detect that a different hand is holding the instrument in a later portion of the video data and update the operating parameters accordingly. In another alternative, the database may include an association between a first user (*e.g.,* the surgeon) and a second user (*e.g.,* the nurse), and detecting the second user holding the instrument can cause the control system 199 to set the operational parameters to those corresponding to the first user rather than the second user.

The final steps 836, 948, 1056 may further include creating and storing usage logs to a database. The usage logs may include information related to the users and instrument involved in an operation over time. In a first example, the usage logs may be stored with respect to each instrument present in the operating room and illustrate when the instrument was used and by who. In a second example, the usage logs may be stored with respect to each user present in the operating room and described which instruments were used by the user and during which time frame. In a third example, the usage logs may be stored with respect to stages of an operation and indicate which tools were used by which user and for how long during each stage of the operation. Combinations of these examples are contemplated. In any case, the usage logs may be configured to be retrieved for review at a later time and may be displayed on one of the displays 116, 117, 118.

Although not discussed in detail above, the user (and the hand thereof) and the end effector may be identified according to various techniques. Starting with the user, in one implementation, the user manually inputs their identify to the control system 199, such as using one of the user interfaces or voice commands. In another implementation, the user may be identified by the navigation system 110 via a nametag worn by the user. In yet another implementation, the user may be identified according to a pattern present on the hand of the user within the cropped image frame, such as a vein pattern and the like. Regarding the end effector, in one implementation, a barcode/RFID tag may be coupled to the end effector and detected by the navigation system 110. In another implementation, the user may manually input the end effector, such as using one of the user interfaces or voice commands. In yet another implementation, the shape/size/etc. of the end effector may be detected via object detection techniques applied to the cropped image frame. Other methods of user and end effector identification are contemplated.

In addition to the combinations already described, the methods 800, 900, 1000 described in this section may be implemented in combination with any of the multi-camera tracking methods 400, 500, 600, 700 described in the previous section, in whole or in part. A number of examples are provided below.

First, the control system 199 may receive first video data from a first video source and second video data from a second video source. For example, during step 804, step 904, or step 1004. After the video data is received, the hand of the user may be detected within the first video data and the second video data, and sub-regions of the first and second video data, each of which include the hand of the user, may be identified. This may occur during steps 812 and 820, steps 912 and 920, or steps 1012 and 1016. Further, the control system 199 may determine a presence of the surgical instrument in both sub-regions and determine at least one characteristic of the surgical instrument (in each sub-region) in response to detecting the presence of the surgical instrument in each sub-region, such as during steps 828 and 832, steps 940 and 944, or step 104. The control system 199 may then control the surgical instrument based on the instrument characteristics determined in each sub-region, such as at step 836, step 948, or step 1056.

Second, the control system 199 may monitor tracking quality associated with each of the video sources being used to track the hand of the user, as well as track based on the tracking quality of each video source. For example, the control system 199 may determine a first tracking confidence associated with the sub-region of the first video data, determine a second tracking confidence associated with the sub-region of the second video data, and track the hand based on the first tracking confidence and the second tracking confidence. More specifically, the control system 199 may define a tracking confidence threshold, compare the first tracking confidence and the second tracking confidence to the tracking confidence threshold. Subsequently, the control system 199 may track the hand in the sub-region of the first video data in response to the first tracking confidence being higher than the tracking confidence threshold, and/or track the hand in the sub-region of the second video data in response to the second tracking confidence being higher than the tracking confidence threshold. These actions may be taken during the entirety of, or as part of any one or more step of, the methods 800, 900, 1000. In one example where the actions are taken during only part of the method 800, 900, 1000, they may be performed at step 816, at steps 916 and/or 936, or at steps 1044. In another example, the steps may be included in the tracking steps, such as at step 824, at step 932, or at step 1028.

Third, the control system 199 may rely on multiple video sources to avoid occlusion issues. For example, the control system 199 may determine that the hand is occluded in the sub-region of the video data captured by a first video source, and switch to tracking the hand in the sub-region of the video data captured by a second video source in response to the hand being occluded in the sub-region of the first video data. As an example, this may be carried out at step 816, at step 916, or at step 1044. As another example, like the actions taken with respect to tracking quality described above, the occlusion-related steps may be included in the tracking steps, such as at step 824, at step 932, or at step 1028.

Fourth, the control system 199 may extract features of the hand of the user with multiple video sources and create a hand tracking model that can be used to track the hand across the various video sources. For example, the control system 199 may extract a first set of hand features associated with the hand of the user from video data captured by a first video source and create a hand tracking model based on the first set of hand features. Further, the control system 199 may extract a second set of hand features associated with the hand of the user from video data captured by a second video source. With the second set of hand feature, the control system 199 may modify the hand tracking model based on the second set of hand features to create an updated hand tracking model, and subsequently track the hand with at least one of the video sources using the updated hand tracking model. In some implementations, the control system 199 may match at least one feature of the first set of hand features to at least one feature of the second set of hand features when creating the hand tracking model. The matching may include identifying which of the second set of hand features correspond to features of the first set of hand features. These actions may be performed during at least one of steps 804-824, steps 904-932, or steps 1004-1028.

Fifth, the control system 199 may extract features of the hand of the user with multiple video sources and create multiple hand tracking models, each model then being used to track the hand within the video data captured by the corresponding video source. For example, the control system 199 may extract a first set of hand features associated with the hand of the user from first video data captured by a first video source, create a first hand tracking model based on the first set of hand features, and track the hand in a sub-region of the first video data using the first hand tracking model. At the same time, the control system 199 may extract a second set of hand features associated with the hand of the user from second video data captured by a second video source, create a second hand tracking model based on the second set of hand features, and track the hand in the sub-region of the second video data using the second hand tracking model. These actions may be performed during at least one of steps 804-824, steps 904-932, or steps 1004-1028.

Sixth, the control system 199 may incorporate sensor data from a sensor unit coupled to the user into the tracking of the hand of the user. For example, the control system 199 may create a hand tracking model based on a first set of hand features extracted from the video data, like in other implementations, and then create an updated hand tracking model based on sensor data received from a sensor unit coupled to the user. Subsequently, the hand may be tracked with the camera using the updated hand tracking model. The sensor unit coupled to the user may be like those described above and include wearable sensors such as inertial measurement units, passive trackers (e.g., radiopaque gloves), active trackers, near-field communication enabled devices, RFID devices, Bluetooth devices, and the like. Similar to other combinations/implementations, these actions may be performed during at least one of steps 804-824, steps 904-932, or steps 1004-1028.

Again, any one of these examples, and/or any one of the multi-camera tracking methods 400, 500, 600, 700, may be combined in whole or in part with the methods 800, 900, 1000 described in this section.

Several implementations have been discussed in the foregoing description. However, the implementations discussed herein are not intended to be exhaustive or limiting. Further, the terminology which has been used is intended to be in the nature of words of description rather than of limitation. Many modifications and variations are possible in light of the above teachings and the systems/methods may be practiced otherwise than as specifically described.

## Claims

1. A surgical system (100) configured to control a surgical instrument (154), the surgical system (100) comprising:
a camera (122, 132, 200); and
a control system (199) coupled to the camera (122, 132, 200) and the surgical instrument (154), wherein the control system (199) is configured to:
receive video data from the camera (122, 132, 200),
detect a hand of a user within the video data,
identify a sub-region (360) of the video data that includes the hand of the user,
track the hand in the sub-region (360) of the video data,
detect a presence of the surgical instrument (154) in the sub-region (360) of the video data,
determine at least one characteristic of the surgical instrument (154) in response to detecting the presence of the surgical instrument (154) in the sub-region (360) of the video data, and
control the surgical instrument (154) based on the at least one characteristic of the surgical instrument (154).

2. The surgical system (100) of claim 1, wherein the control system (199) is further configured to utilize a hand detection algorithm (300) to detect the hand of the user within the video data and to track the hand of the user in the sub-region (360).

3. The surgical system (100) of claim 2, wherein the control system (199) is configured to:
utilize the hand detection algorithm (300) to identify the sub-region (360);
generate a bounding region within or surrounding the sub-region (360) of the video data;
apply the hand detection algorithm (300) within the bounding region; and
disregard or remove portions of the video data that are outside of the bounding region.

4. The surgical system (100) of claim 3, wherein the control system (199) is further configured to:
segment the video data into a first set of pixels corresponding to the hand of the user and a second set of pixels corresponding to the surgical instrument (154); and
generate the bounding region to surround the first set of pixels and the second set of pixels.

5. The surgical system (100) of claim 3 or 4, wherein the control system (199) is further configured to:
determine a range of motion of the hand from the video data; and
generate the bounding region to encompass the range of motion of the hand.

6. The surgical system (100) of any one of claims 2-5, wherein the control system (199) is further configured to:
apply the hand detection algorithm (300) to identify a hand region in the video data that includes the hand of the user;
apply an instrument-in-hand detection algorithm (330, 332, 334, 336, 338) to the video data to detect the surgical instrument (154) and identify an instrument region in the video data that includes the surgical instrument (154); and
identify when the sub-region (360) of the video data includes the hand region and the instrument region.

7. The surgical system (100) of claim 6, wherein the control system (199) is further configured to:
classify an interaction between the hand region and the instrument region in the sub-region (360); and
control the surgical instrument (154) based on the at least one characteristic of the surgical instrument (154) and the classified interaction.

8. The surgical system (100) of claim 6 or 7, wherein the control system (199) is further configured to apply a primary hand detection algorithm (310, 312, 314, 316, 318) to the video data to detect whether the hand region reflects a primary hand of the user, wherein the primary hand detection algorithm (310, 312, 314, 316, 318) detects whether the hand region reflects the primary hand of the user by being configured to evaluate interactions of both hands of the user with respect to the surgical instrument (154).

9. The surgical system (100) of any preceding claim, wherein the at least one characteristic of the surgical instrument (154) comprises one or more of:
a physical characteristic of the surgical instrument (154) including at least one of:
a geometry of the surgical instrument (154);
an appearance of the surgical instrument (154); and
a physical configuration of the surgical instrument (154);
an operational characteristic of the surgical instrument (154) including at least one of:
an operational status of the surgical instrument (154);
a mode of operation of the surgical instrument (154); and
an operational parameter of the surgical instrument (154); and/or
a procedural context characteristic of the surgical instrument (154) including at least one of:
a type of the surgical instrument (154);
an identification of the surgical instrument (154);
an accessory attached to the surgical instrument (154);
an interaction of the surgical instrument (154) with an anatomy; and
a procedural stage in which the surgical instrument (154) is used.

10. The surgical system (100) of any preceding claim, wherein the at least one characteristic of the surgical instrument (154) comprises a hand interaction context characteristic relating to a context of interaction between the hand and the surgical instrument (154).

11. The surgical system (100) of claim 10, wherein the hand interaction context characteristic of the surgical instrument (154) includes at least one of:
the surgical instrument (154) being held by the hand;
the surgical instrument (154) being held by a primary hand;
the surgical instrument (154) being held by a secondary hand;
the surgical instrument (154) being held by both primary and secondary hands;
a position and/or orientation of the surgical instrument (154) when held by the hand;
a change of pose or movement pattern of the surgical instrument (154) when held by the hand;
a duration that the surgical instrument (154) is held by the hand;
the surgical instrument (154) not being held by any hand;
a position and/or orientation of the surgical instrument (154) when not held by the hand;
a duration that the surgical instrument (154) is not held by any hand;
an action applied to the surgical instrument (154) by the hand;
the surgical instrument (154) being picked up by the hand;
the surgical instrument (154) being placed down by the hand; and
a spatial relationship or proximity between the hand and the surgical instrument (154).

12. The surgical system (100) of any preceding claim, wherein the control system (199) controls the surgical instrument (154) based on the at least one characteristic by being configured to perform one or more of:
activate the surgical instrument (154);
deactivate the surgical instrument (154);
adjust operational parameters of the surgical instrument (154);
configure a mode of operation of the surgical instrument (154);
configure the surgical instrument (154) based on user-specific preferences;
link the surgical instrument (154) to a control device (152B, 156) that controls the surgical instrument (154); and
unlink the surgical instrument (154) from a control device (152B, 156) that control the surgical instrument (154).

13. The surgical system (100) of any preceding claim, wherein:
the video data includes a plurality of image frames; and
the control system (199) is further configured to:
detect the hand of the user within each of the plurality of image frames;
identify a plurality of sub-regions (360), each sub-region (360) corresponding to a portion of one of the plurality of image frames that includes the hand of the user;
detect the presence of the surgical instrument (154) in each of the sub-regions (360); and
determine the at least one characteristic of the surgical instrument (154) in response to detecting the presence of the surgical instrument (154) in the sub-regions (360).

14. A computer-implemented method of operating at least the control system (199) of the surgical system (100) of any preceding claim.

15. A computer program product comprising instructions, which when executed by one or more processors, are configured to implement the operations of at least the control system (199) of the surgical system (100) of any of one of claims 1-13.
